(19) Europäisches Patentamt
European Patent Office
Office européen des brevets

(11) EP 2 366 386 A1

(12) **EUROPEAN PATENT APPLICATION**
published in accordance with Art. 153(4) EPC

(43) Date of publication:
**21.09.2011 Bulletin 2011/38**

(21) Application number: **09823117.8**

(22) Date of filing: **26.10.2009**

(51) Int Cl.:
***A61K 9/51*** *(2006.01)* ***A61K 47/36*** *(2006.01)*
***A61K 47/18*** *(2006.01)*

(86) International application number:
**PCT/ES2009/070461**

(87) International publication number:
**WO 2010/049562 (06.05.2010 Gazette 2010/18)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.10.2008 ES 200803135**
**28.10.2008 ES 200803136**
**30.03.2009 ES 200900916**

(71) Applicant: **Universidade De Santiago De Compostela**
**15782 Santiago de Compostela (ES)**

(72) Inventors:
- **SÁNCHEZ BARREIRO, Alejandro** **(ES)**
- **SEIJO REY, Begoña** **(ES)**
- **PAOLICELLI, Patrizia** **(ES)**
- **KONAT ZORZI, Giovanni** **(ES)**
- **PÁRRAGA MENESES, Jenny** **(ES)**

(74) Representative: **ABG Patentes, S.L.**
**Avenida de Burgos 16D**
**Edificio Euromor**
**28036 Madrid (ES)**

(54) **NANOPARTICULATE SYSTEMS PREPARED FROM ANIONIC POLYMERS**

(57) The present invention relates to a system for administering active ingredients comprising nanoparticles having an average size of less than 1 micrometer in turn comprising: (a) at least one anionic polymer; (b) a cationic cross-linking agent; and optionally (c) a cationic polymer; **characterized in that** the nanoparticles are cross-linked by means of electrostatic type interactions. Additionally, the invention relates to pharmaceutical, cosmetic, personal hygiene and nutritional compositions comprising said nanoparticle system, as well as to methods for the preparation and uses thereof.

FIGURE 1

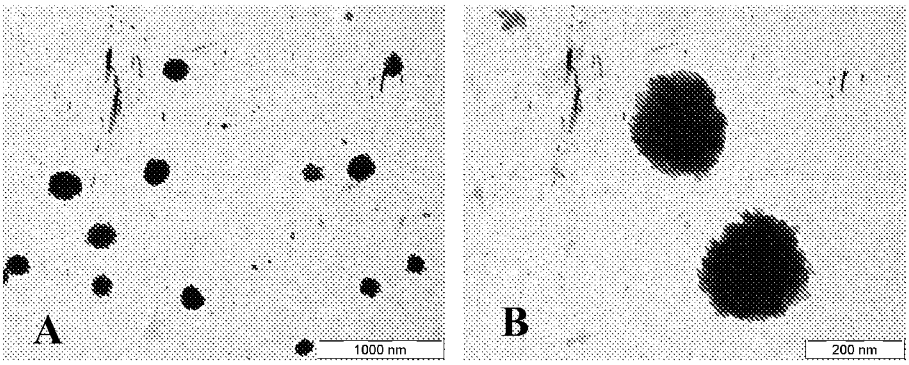


EP 2 366 386 A1

## Description

### Field of the Invention

The present invention relates to the development of nanoparticulate systems useful in the administration of active ingredients. More specifically, the invention relates to nanoparticulate systems comprising a polymer or a mixture of polymers provided with negative electric charge and a molecule or a mixture of low molecular weight molecules with a positive charge capable of acting as ionic cross-linking agents for the previous polymers without establishing chemical bonds with them. The invention additionally relates to pharmaceutical, cosmetic and nutritional compositions comprising them as well as to methods for the preparation thereof.

### Background of the Invention

Nanotechnology in general, and nanoparticulate systems more specifically, present a huge potential that is clearly recognized in several fields (UNESCO, The ethics and politics of nanotechnology, Division of Ethics of Science and Technology, UNESCO Ed., Paris, 2006), having awakened a great interest above all in the biomedical field (U.S. Food and Drug Administration. Nanotechnology, A Report of the U.S. Food and Drug Administration Nanotechnology Task Force, FDA Ed., Rockville, MD, July 2007), (WHO, Initiative for Vaccine Research of the Department of Immunization, Vaccines and Biologicals, WHO/IVB/06.03, WHO Ed., Geneva, Switzerland, April 2006). Despite the aforementioned, the nanoparticulate systems developed up until now have not provided an answer to the expectations initially placed on them. Therefore, the general idea is that it is necessary to develop new systems capable of meeting the challenge of making suitable use of their recognized potential (M. Friede and M.T. Waterdo, Advanced Drug Delivery Reviews, 57, 2005, 325-31); (T.G. Park, J.H. Jeong, S.W. Kim, Advanced Drug Delivery Reviews, 58, 2006, 467-486).

There are various causes for the limitations set forth above. By considering the specific case of nanoparticles based on chitosan, a polymer which is widely mentioned in literature as being indispensable for forming the nanoparticles by ionic cross-linking, the absence of added value for systems of this type of in comparison to simpler formulations has been alluded to recently. Specifically, the results presented by some works question the supposed versatility and potential of chitosan nanoparticles since no significant differences are found when comparing it to simple solutions of the bioactive molecule and said polymer (A. M. Dyer, M. Hinchcliffe, P. Watts, J. Castile, 1. Jabbal-Gill, R. Nankervis, A. Smith, and L. Illum, Pharm. Res., 19, 2002, 998-1008). In addition, the cytotoxicity associated with said chitosan nanoparticles which has been directly related to the surface electric charge of these systems has recently been pointed out (B. Loretz and A. Bernkop-Schnürch, Nanotoxicology, 1, 2007, 139-148). Toxicity results of this type especially concern regulatory agencies such as the FDA which believes that it is important to not lose sight of aspects such as the important positive charge associated with some nanoparticulate systems (U.S. Food and Drug Administration. Nanotechnology, A Report of the U.S. Food and Drug Administration Nanotechnology Task Force, FDA Ed., Rockville, MD, July 2007). It is obvious however that the advantages or limitations of a nanoparticulate system do not derive exclusively from a single characteristic such as its surface charge but rather from a set of characteristics among which, in addition to the surface charge, the actual nature of the components used in the preparation of said nanoparticles must also be taken into account. As an illustrative example, the mucoadhesive character and the capacity for interacting with the mucosal surfaces of nanoparticles prepared from a polymer such as chitosan have been related exclusively to the cationic nature of this polymer and the positive surface charge of the systems based on the use thereof. However, the surface charge cannot be considered as the only factor responsible for such behavior or properties since they are not observed to the same extent when other also cationic polymers are used. In fact, a previous study has been able to demonstrate how nanoparticulate systems coated with cationic polymers such as polylysine and chitosan present drastically different behaviors after their *in vivo* administration despite having a similar net surface charge (Calvo P, Vila-Jato JL and Alonso MJ; Int J Pharm., 153, 1997, 41-50). Therefore, it seems logical to think that the actual nature of the components of nanoparticulate systems of this type together with their physicochemical characteristics determine their behavior and, therefore, their potential as has recently been indicated (Moreau et al., Journal of Drug Targeting, 10, 2002, 161-173).

Considerations such as those set forth above have recently prompted interest in investigating the application of nanotechnology to new materials and thus developing new nanoparticulate systems (U.S. Food and Drug Administration, FDA Consumer magazine, FDA Ed., November-December 2005 Issue, 2005). This interest is more patent in the case of nanosystems intended for systemic administration where the toxicity problems and/or adverse effects or unwanted effects associated with the surface charge or the actual characteristics of the materials used until now in their development take on special importance. In fact, although a system with a positive net charge may be of great interest as a carrier for topical administration, that positive charge may also be a problem when it is administered through the systemic route since it will give rise, without a doubt, to hemagglutination and other adverse effects related to interaction with natural components of the organism (Kainthan et al., Biomaterials 27, 2006, 5377-5390). Possibly due to that, many experts in the field of gene therapy have even predicted that the development of new carriers is a field of work which

will be prolonged for the next 35 years (N. Blow, Nature, 450, 2007, 1117-1120), making special mention of the limitations which have been referred to for the development of carriers for systemic administration.

Up until now various materials have been used to formulate nanoparticulate systems, many of which have been capable of acting as carriers for administering drugs or genetic material. However, although nanoparticle systems are mentioned in many cases, it is necessary to remember that such designation may encompass two types of clearly different systems in terms of preparation technique, structure, capacity for associating and releasing molecules, versatility and potential. These systems, clearly differentiated in the literature (J.K. Vasir and V. Labhasetwar, Expert Opinion on Drug Delivery, 3, 2006, 325-344) (Q. Gana, T. Wang, C. Cochrane, P. McCarron, Colloids and Surfaces B: Biointerfaces 44, 2005, 65-73), are the following:

- Nanoparticulate complexes established between positively charged materials and a bioactive molecule with negative net charge such as a nucleic acid derivative, for example, the high density of amino groups present in the chitosan backbone allows complexing plasmids DNA having negative charge, giving rise to the formation of self-assembled complexes between both components in a spontaneous but non-controlled manner. These complexes are obtained without being able to control properties as important as the size or the surface charge thereof since the formation of this type of particles is merely due to the tropism established between two molecules having an opposite charge. In fact, without the bioactive molecule with negative net charge, it would not be possible to obtain such nanosystems. Therefore, it is not possible to develop nanoparticles of this type which are blank or in which said molecule is not loaded.
- Nanoparticles prepared from cross-linked polymers. Cross-linking is a controlled process which allows obtaining homogenous, adjustable and reproducible nanoparticles having predetermined size and surface charge. The cross-linking process can be chemical or ionic. The first of said processes is based on the formation of stabilizing covalent bonds due to the use of agents from the aldehyde group which are characterized by their toxicity and by not being accepted for use in humans. Furthermore, agents of this type may also give rise to the cross-linking and inactivation of the bioactive molecule itself that is to be associated with the system, especially if they are molecules with amino groups as in the case of peptides and proteins. All these problems of aldehydes and chemical cross-linking agents are described in the literature.

In contrast, the ionic cross-linking technique, also known as ionic or ionotropic gelling is characterized by its mildness and by being reversible. This technique has traditionally been developed between a cationic macromolecule and a polyanion, giving rise to the formation of systems which, unlike the complexes, are characterized by being matrix structures in which the associated bioactive molecule is completely or partially trapped inside the constitutive polymer matrix thereof and generated in the ionotropic cross-linking process. This polymer matrix is obtained as a result of the inter- and intra-molecular ionic bonds between the polyanion and the cationic macromolecule which spontaneously gels under nanoparticulate form. This formation mechanism provides, as an added value with respect to the complexes, a protection for the bioactive molecule against external medium which complexes are not able to provide in the same extent. Therefore, a fast, economical, easily reproducible, and scalable technique which requires a very simple technology, all being aspects which are undoubtedly of interest for the industry, is provided.

The ionic cross-linking technique has been described for the formation of chitosan nanoparticles, chitosan being a cationic molecule which cross-links with the tripolyphosphate polyanion. However, the aforementioned limitations for systems of this type which include chitosan in their composition have prompted many inventors to develop systems in which chitosan is combined with different anionic macromolecules, such as hyaluronic acid, for example, but the presence of chitosan for the formation thereof has been always required.

Other materials which have also been used in the state of the art for obtaining nanoparticle systems comprise dextrans, carrageenan and polyarginine.

Thus, documents WO2005021044 and US20077155658 describe systems smaller than 200 nm which need the use of carbohydrates capable of complexing the genetic material to be associated in a first phase and subsequently the addition of polyarginine.

Documents US 6,565,873 and US 7,053,034 describe nanoparticles the formation of which requires the use of fatty materials.

Documents US 2005/0266090 A1 and US 2005/0008572 A1 describe the formation of core-shell (core-coat or onion-like) systems formed by two different parts: a core polymer and a corona polymer having a different composition surrounding said core. Said structures are the result of applying a technique in which the constitutive polymers are sequentially added and in which it is necessary to use, among others, steps for atomizing the solutions (Propok *et al.*, 2001; Prokop *et al.*, 2002).

In addition, the techniques used for the formation of nanoparticles and nanoparticulate systems are generally complex and require determined compositions affecting the properties and characteristics thereof. Document WO 2001/9620698 A1 describes nanoparticles obtained by an emulsification methodology making the use of organic solvents

necessary. The use of said solvents entails a series of risks perfectly known by the industries for giving rise to a special concern by the regulatory agencies.

The nanoparticles described in document US 2005/0008572 A1 containing a type of dextrans (polyaldehyde dextrans) need, for the formation thereof, to establish a covalent bond with said component so that the dextrans are formed, finally leading to the formation of a different chemical entity.

Document US 6,383,478 B1 relates to nanoparticles in which the obliged incorporation of at least two polyanions in addition to one or more small cations is necessary in their preparation. Ultimately, they are systems with a significant degree of complexity in terms of their composition.

Document US 7,045,356 describes multilayer nanoparticles for the formation of which it is necessary to establish conditions such that they allow the formation of intermolecular bonds between the polymers.

Document US 6,916,490 relates to microparticles coarcevate systems which require chemical cross-linking between the polymers for their formation.

Documents US 6,919,091 and 7,098,032 describe nanoparticle systems in which the nanoparticles are smaller than 100 nanometers for the formation of which it is necessary to carry out three steps: (1) complexing the genetic material to be associated; (2) complexing a second polymer; (3) final ionic cross-linking to ensure the integrity of the system.

Documents US 6,475,995 and 7,344,887 describe nanostructures produced by electrodeposition or by coacervation, the suggested polycations being gelatin or chitosan.

In view of the documents of the state of art and of the drawbacks presented by current nanoparticulate systems in terms of the composition, toxicity and method for obtaining them, there is therefore a need for developing nanoparticulate systems from low toxicity biocompatible materials and reagents which provide greater control in the physicochemical properties of the nanoparticles and which may be obtained by means of simple and efficient methods.

## Brief Description of the Invention

The present inventors have discovered that, a nanoparticulate system easily obtained by means of an ionic gelling method where the nanoparticles comprise a cross-linked anionic polymer in the presence of a cationic cross-linking agent, allows an efficient association of bioactive molecules and the subsequent release into the suitable medium, which release may be controlled release by means of selecting the components of the nanoparticles. Said nanoparticles have the additional feature of not presenting toxicity and of being stable in biological mediums, further preventing the degradation of the molecules associated therewith.

Thus, in a first aspect the invention relates to a system for administering bioactive molecules comprising nanoparticles having an average size of less than 1 micrometer, comprising:

a) at least one anionic polymer;
b) a cationic cross-linking agent; and optionally
c) a cationic polymer;

characterized in that the nanoparticles are cross-linked by means of electrostatic type interactions.

In another aspect, the invention relates to a pharmaceutical composition comprising a system as has been previously defined.

In an additional aspect, the invention relates to a cosmetic composition comprising a system as has been previously defined.

In another aspect, the invention relates to a personal hygiene composition.

In another aspect, the invention relates to a nutritional composition comprising a system as has been previously defined.

In another aspect, the invention relates to a composition intended for diagnosis comprising a system as has been previously defined.

In another aspect, the invention relates to a method for preparing a system as has been previously defined which comprises:

a) preparing an aqueous solution of at least one anionic polymer;
b) preparing an aqueous solution of a cationic cross-linking agent and optionally adding therein a cationic polymer;
c) mixing the solutions obtained in a) and b) under stirring with spontaneous formation of the nanoparticles.

In a particular embodiment, the optional cationic polymer is added to the nanoparticles once formed.

The invention also relates to the use of a system as has been previously defined in the preparation of a medicinal product. In a particular embodiment, said medicinal product is for application in gene therapy, gene silencing or genetic

interference or genetic vaccination.

In an additional aspect, the invention relates to the use of a system as has been previously defined for manipulating or altering the biological characteristics of living cells including autologous cells, allogeneic cells, xenogeneic cells or cell cultures and for subsequently using said cells or cell groups to obtain a therapeutic effect, diagnostic effect, preventive effect or for regenerative purposes, or for modifying the production of compounds by said cells.

In another additional aspect, the invention relates to the use of a system as has been previously defined for modifying, correcting or introducing organoleptic properties or improving the stability in a medicinal product or in a cosmetic product.

A final aspect of the invention relates to the use of a system as has been previously defined for conditioning, modifying or restoring the characteristics of water, foods or nutritional supplements, as well as for modifying, correcting or introducing new organoleptic properties or improving the stability thereof and for facilitating or making the administration of foods or nutrients to living beings possible.

## Brief Description of the Drawings

Figure 1 shows the nanoparticles prepared from colominic acid associating effectively with a bioactive molecule and having a regular spherical shape and a homogenous nanometric particle size: TEM images of nanoparticulate systems prepared from colominic acid associating genetic material (DNA plasmid).

Figure 2 shows the nanoparticles associating effectively with siRNA: Image from 1% agarose gel electrophoresis loaded with: free siGAPDH, siGAPDH associated with the nanoparticles prepared from chondroitin sulfate (A) or siGAPDH associated with the nanoparticles prepared from hyaluronic acid (B).

Figure 3 shows the nanoparticles prepared from hyaluronic acid associating a bioactive molecule having a regular spherical shape and a nanometric size. TEM images of nanoparticulate systems prepared from hyaluronic acid associating interfering RNA siGAPDH.

Figure 4 shows the nanoparticles prepared from hyaluronic acid associating a bioactive molecule for cosmetic use having a regular spherical shape and a nanometric size. TEM images of nanoparticulate systems prepared from hyaluronic acid associating kinetin.

Figure 5 shows the nanoparticles prepared from chondroitin sulfate associating a bioactive molecule of cosmetic use having a regular spherical shape and a nanometric size. TEM images of nanoparticulate systems prepared from chondroitin sulfate associating kinetin.

Figure 6 shows that the developed nanoparticles release the bioactive molecule for associated cosmetic use and it is possible to control said release by conveniently selecting the components of said nanoparticles: Study of kinetin release from nanoparticles prepared using hyaluronic acid (A) or chondroitin sulfate (B).

Figure 7 shows it is possible to lyophilize and resuspend the nanoparticles without altering them: The size variation index for the nanoparticles subjected to a lyophilization process is not modified when the nanoparticles are lyophilized at a concentration of 0.5 mg/ml in the presence of 5% glucose (lyophilization in the presence of 5% trehalose: white blocks; lyophilization in the presence of 5% glucose: black blocks) (Df/Di = Ratio between the average particle size of the formulation before its lyophilization and the average size after the lyophilization and subsequent resuspension of the formulation in milliQ water), (n=3).

Figure 8 shows the nanoparticulate systems based on chondroitin sulfate associating kinetin not presenting cytotoxicity in fibroblasts: Values of cell viability obtained by means of the XTT test using untreated cells as negative control (0% cell death).

Figure 9 shows the nanoparticulate systems based on chondroitin sulfate associating kinetin being effectively internalized in fibroblasts: Fluorescence confocal microscopy image of nanoparticles labeled with fluoresceinamine (green glow) internalized in fibroblasts the cytoskeleton of which has been stained with Bodipy (red glow). Sections in the x-y axis are shown in the central box and sections in the corresponding x-z axes are shown in the side images.

Figure 10 shows the effective biological response (GAPDH silencing) in human cornea cells by using nanoparticles prepared from chondroitin sulfate associating interfering RNA (siGAPDH) and with surface electric charge modulated by means of adding a cationic polymer. Negative controls used: Untreated cells and cells treated with nanoparticles associating a non-specific siRNA.

## Detailed Description of the Invention

The present invention relates to the preparation of nanoparticulate systems for administering, among others, biologically active molecules, comprising nanoparticles having an average size of less than 1 micrometer, wherein said nanoparticles comprise at least one anionic polymer; a cationic cross-linking agent; and optionally a cationic polymer;

characterized in that the nanoparticles are cross-linked by means of electrostatic type interactions.

In the present invention, the term "nanoparticles" refers to stable structures having homogenous, reproducible and modulable characteristics that can be perfectly differentiated from self-assembled systems which are formed as a consequence of a controlled ionotropic cross-linking process of the constitutive anionic polymer thereof mediated by cationic cross-linking agents. The electrostatic interaction which occurs between the different components of the nanoparticles in the cross-linking process generates characteristic physical entities which are independent and observable, the average size of which is less than 1 μm, i.e., an average size of between 1 and 999 nm.

The term "average size" is understood as the average diameter of the nanoparticle population comprising the cross-linked polymer structure which moves together in an aqueous medium. The average size of these systems can be measured using standard methods known by the person skilled in the art.

The nanoparticles of the system of the invention have an average particle size of less than 1 μm, i.e., they have an average size of between 1 and 999 nm, preferably of between 50 and 800 nm. The average particle size is mainly influenced by the composition and the conditions for particle formation.

In addition, the nanoparticles may have an electric charge (measured by means of the Z potential), the magnitude of which may have positive or negative values depending on the proportion of the different components in the system. In a particular embodiment of the invention, the nanoparticles have a negative charge ranging between -1 mV and -40 mV.

The zeta potential of the particle of the systems of the invention can be measured using standard methods known by the person skilled in the art which are described, for example, in the experimental part of the present specification.

Anionic polymer

The term "anionic polymer" is understood as any polymer, preferably of a natural origin with a negative net charge, including in said definition those anionic polymers on which modifications such as enzymatic or chemical fragmentation or derivatization have been performed. In a particular embodiment, the anionic polymer is selected from hyaluronic acid or salts thereof, colominic acid or derivatives, chondroitin sulfate, keratan sulfate, dextran sulfate, heparin, carrageenan, glucomannan, as well as fragments or derivatives thereof.

<u>Hyaluronic acid</u>

Hyaluronic acid or hyaluronan is a glycosaminoglycan widely distributed throughout connective, epithelial and neural tissues. It is one of the main components of the extracellular matrix and it generally contributes significantly to cell proliferation and migration.

• hyaluronan is a linear polymer comprising the repetition of a disaccharide structure formed by the alternating addition of D-glucuronic acid and D-N-acetylglucosamine bound by alternating beta-1,4 and beta-1,3 glycosidic bonds as shown in the following formula:

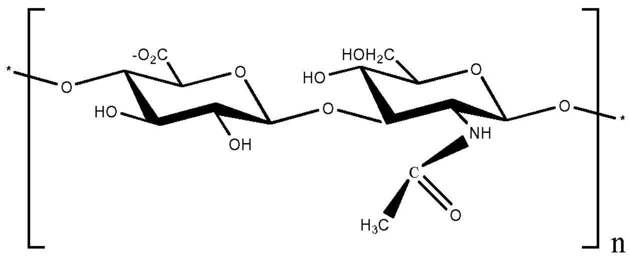

wherein the integer n represents the degree of polymerization, i.e., the number of disaccharide units in the hyaluronan chain.

Hyaluronic acid with a wide range of molecular weights can be used in the context of the present invention. High molecular weight hyaluronic acid is commercially available, whereas low molecular weight hyaluronic acid can be obtained by means of fragmenting the hyaluronic high molecular weight acid using a hyaluronidase enzyme, for example.

The term "hyaluronic, hyaluronic acid, hyaluronan" as used herein includes either hyaluronic acid or a conjugated base thereof (hyaluronate). This conjugated base can be an alkaline salt of hyaluronic acid including inorganic salts such as, for example, sodium salt, potassium salt, calcium salt, ammonium salt, magnesium salt, aluminium salt and lithium salt, organic salts such as basic amino acid salts at neutral pH, said salts are preferably pharmaceutically acceptable. In a preferred embodiment of the invention, the alkaline salt is the sodium salt of hyaluronic acid.

Colominic acid

Colominic acid is a natural polymer of bacterial origin belonging to the family of polysialic acids. It is a linear polymer formed by N-acetylneuraminic acid residues (Neu5Ac; also known as sialic acid), a natural constituent of cells and tissues, bound by glycosidic α-(2→8) bonds. Each N-acetylneuraminic acid residue has a carboxyl group responsible for the negative charge of colominic acid as shown in the following formula:

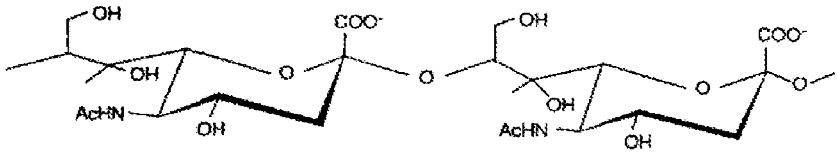

It is undoubtedly a material of interest in the pharmaceutical and cosmetic field as it is biocompatible and biodegradable, and non-immunogenic, the degradation products of which are not toxic (Gregoriadis G et al. Cell. Mol. Life Sci. 2000, 57, 1964-1969). In addition, polysialic acids are characterized by having, among other properties, a very long plasma half-life, therefore they have been proposed as the alternative to polyethylene glycol derivatives to prolong the residence time of drugs and release systems for bioactive molecules, such as liposomes, in plasma. In fact, patent "WO/2008/033253 - Liposome complexes containing pharmaceutical agents and methods" resorts to using them to superficially modify preformed liposomes. Finally, taking into account its structural characteristics, this material offers the possibility of modifying it, for example by introducing amino groups and the resulting cationization.

Dextran sulfate

Dextran sulfate is a complex glucan (polysaccharide) formed by units of glucose molecules each of which contains approximately two sulfate groups as shown in the following formula:

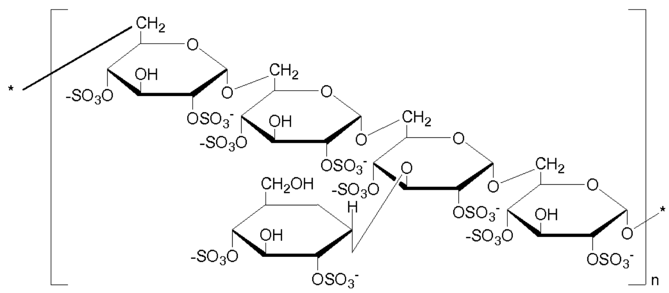

Dextran sulfate is prepared by means of dextran sulfation and subsequent purification by means of methods well-known by the person skilled in the art.

Heparin

Heparin is a naturally occurring substance from the family of glycosaminoglycans the chemical structure of which comprises the repetition of 2-O-sulfated-α-L-iduronic acid and 2-deoxy-2-sulfamido-α-D-glucopyranosyl-6-O-sulfate disaccharide monomer units depicted below:

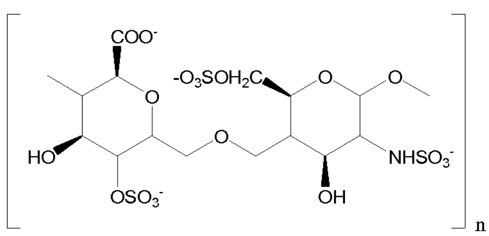

wherein n is an integer and represents the degree of polymerization, i.e., the number of monomer units in the heparin chain.

It is possible to use both fractionated and non-fractionated heparin in the context of the present invention. Traditional or unfractionated heparin is clearly distinguished from fractionated or low molecular weight heparin. The first of them is a natural substance present in all vertebrates. Both types of heparin can be used in the form of free base or in the form of salt, such as for example the sodium or calcium salt thereof.

Fractionated or low molecular weight heparin is produced by chemical or enzymatic depolymerization of conventional heparins. Examples of heparins of this type are enoxaparin, parnaparin, dalteparin and nadroparin, as well as their salts such as the sodium and calcium salts.

Heparin derivatives can also be used in the composition of the nanoparticles of the present invention. These derivatives are known in the state of the art and they arise as the consequence of the reactivity of the different functional groups present in the molecule. Thus, N-acetylated heparin, O-decarboxylated heparin, oxidized heparin or reduced heparin are widely known.

<u>Chondroitin sulfate</u>

Chondroitin sulfate is a sulfated glycosaminoglycan (GAG) made up of a chain of alternating sugars. It is normally bound to proteins as part of a proteoglycan. It is represented by means of the following structure:

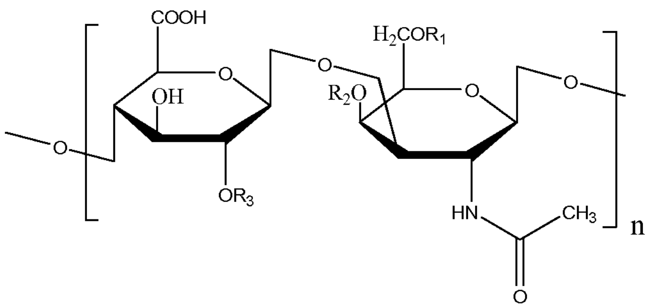

wherein n is an integer and represents the degree of polymerization, i.e., the number of disaccharide units in the chondroitin sulfate chain and wherein $R_1$, $R_2$ and $R_3$ are independently a hydrogen or a $SO_3H$ group. Each monosaccharide can be left without being sulfated, sulfated once, or sulfated twice. The sulfation is mediated by specific sulfotransferases.

In the context of the present invention, the term chondroitin sulfate" includes all its different isomers and derivatives, as well as combinations thereof.

In a particular embodiment, the chondroitin sulfate is selected from the following substances and combinations thereof:

- chondroitin sulfate A which is also known as chondroitin-4-sulfate ($R_1$=H, $R_2$=$SO_3H$ and $R_3$=H) is predominantly sulfated on carbon 4 of the N-acetylgalactosamine (GalNAc) sugar
- chondroitin sulfate B which is also known as dermatan sulfate. This substance is made up of linear repeating units containing N-acetylgalactosamine and either L-iduronic acid or glucuronic acid, and each disaccharide can be sulfated once or twice.
- chondroitin sulfate C which is also known as chondroitin-6-sulfate ($R_1$=$SO_3H$, $R_2$=H and $R_3$=H) is predominantly sulfated on carbon 6 of the GalNAc sugar;
- chondroitin sulfate D which is also known as chondroitin-2,6-sulfate ($R_1$=$SO_3H$, $R_2$=H and $R_3$= $SO_3H$) is predominantly sulfated on carbon 2 of glucuronic acid and on carbon 6 of the GalNAc sugar;
- chondroitin sulfate E which is also known as chondroitin-4,6-sulfate ($R_1$=$SO_3H$, $R_2$= $SO_3H$ and $R_3$=H) is predominantly sulfated on carbon 4 and carbon 6 of the GalNAc sugar;

The term "chondroitin sulfate" also includes the organic and inorganic salts thereof. Such salts are generally prepared, for example, by means of reacting the base form of this compound with a stoichiometric amount of the suitable acid in water or in an organic solvent or in a mixture of the two. Generally, nonaqueous mediums such as ether, ethyl acetate, ethanol, isopropanol or acetonitrile are preferred. Examples of inorganic salts include, for example, sodium salt, potassium salt, calcium salt, ammonium salt, magnesium salt, aluminium salt and lithium salt, and the organic salts include, for example, salts of ethylenediamine, ethanolamine, *N,N*-dialkyl-ethanolamine, triethanolamine, glucamine and basic amino acids. The salts are preferably pharmaceutically acceptable.

The functions of chondroitin depend largely on the properties of the proteoglycan of which it is a part. These functions can be broadly divided into regulatory and structural roles. However, this division is not absolute and some proteoglycans may have both structural and regulatory roles.

With respect to its structural role, chondroitin sulfate is a main component of the extracellular matrix and it is important for maintaining the structural integrity of the tissue. By being part of an aggrecan, chondroitin sulfate is a main component of cartilage. The highly charged and tightly packed sulfate groups of chondroitin sulfate generate electrostatic repulsions providing much of the resistance of cartilage to compression.

Jeratan sulfate is a sulfated glycosaminoglycan similar to chondroitin sulfate in which the sulfate group is in the glucuronic acid.

Carrageenan

Carrageenan or carrageenin is formed by sulfated or non-sulfated galactose and/or anhydrogalactose units bound by alternating α-1,3 and β-1,4 bonds. Depending on the degree of sulfation of the positions of the sulfate groups and on the presence of anhydrogalactose groups, various types of carrageenin with properties such as clearly different hydrocolloids are distinguished. The greater the proportion of sulfate groups, the higher the solubility, and the greater the proportion of anhydrogalactose groups the lower the solubility. All types of carrageenan are included in the context of the present invention. Some of these include, for example, kappa, iota and lambda (k, i and 1) carrageenan.

Glucomannan

Glucomannan is a naturally occurring water-soluble polysaccharide. The chemical structure of this compound consists of a linear polymer chain with a small degree of branching. Specifically, it is formed by D-mannose and D-glucose units bound by β-1,4 bonds at a proportion of 1.6:1, respectively.

In a particular embodiment of the invention, the glucomannan used is a glucomannan derivative with negative charge selected from the phosphorylated derivatives, carboxymethyl and dicarboxy-glucomannan.

Cross-linking agent

The nanoparticles of the invention are characterized by being formed through an ionic interaction mechanism causing the overall precipitation of the components of said nanoparticles in the form of nanoclusters as a consequence of the addition of a cross-linking agent with a positive charge. In addition to being a simple method, it does not require the use of organic solvents or of toxic auxiliary substances. The presence of the cationic cross-linking agent allows the cross-linking of the anionic polymer, and where appropriate, the cross-linking of the latter with the optional cationic polymer by means of an ionic gelling process causing the spontaneous formation of the nanoparticles. Nanoparticles with a size, surface electric charge and structural characteristics which making them suitable as systems for administering active molecules are thus obtained.

In a particular embodiment, the cross-linking agent is an amine of formula $H_2N\text{-}[(CH_2)_x\text{-}NH\text{-}(CH_2)_{and}]_z\text{-}NH_2$, wherein *x*, *y* and *z* independently have a value comprised between 1 and 66. Preferably, *x*, *y* and *z*, independently have a value comprised between 1 and 10.

More preferably, the amine is selected from spermine, spermidine and the salts thereof. These amines are natural components of the cells and body fluids and play a fundamental role in cell proliferation and differentiation processes and in biological macromolecule synthesis processes. Their capacity for inhibiting oxidative stress in living beings and for promoting their longevity has also recently been described (Eisenberg et al., Nature Cell Biology, 4 October 2009, doi:10.1038/ncb1975). Although cells are capable of synthesizing the amines they need for cell growth processes, cellular internalization mechanisms which allow obtaining these amines from the blood stream have been described. These mechanisms are influenced by proteoglycans such as chondroitin sulfate and hyaluronic acid (Belting M. et al. Biochem J 1999, 338, 317-323). Therefore, it seems logical to assume a synergistic effect between the constituents of the nanoparticles object of the present invention and the cross-linking agent used in their preparation.

In a particular embodiment, the cross-linking agent/anionic polymer ratio by weight is comprised between 0.1/1 and 0.5/1, preferably between 0.2/1 and 0.4/1, which provides formulations with a low polydispersity.

Cationic polymer

In a particular embodiment of the invention, the nanoparticles forming the system can optionally comprise a polymer with a positive charge for the purpose of modulating the characteristics of nanoparticulate systems which have greater importance in their interaction with biological mediums, such as particle size, surface electric charge and composition, and thus providing them with a greater versatility.

In the context of the present invention, "cationic polymer" is understood as any polymer, preferably of a natural origin, with a positive net charge. In a particular embodiment, the cationic polymer is selected from cationized dextrans, polyamino acids such as polylysine or polyarginine, modified proteins such as gelatin, collagen and atelocollagen or the cationized derivatives thereof.

"Cationized dextran" and "modified proteins such as cationized gelatins, collagens or atelocollagens" are understood as the previous molecules modified such that amine groups conferring them a greater cationic character from than that it may have without modification, are introduced.

The nanoparticles of the present invention provide systems with a high capacity for associating bioactive molecules. Therefore, in an additional aspect the invention relates to a system as has been previously defined further comprising a bioactive molecule. The release of the bioactive molecules can be controlled by means of selecting the components of the nanoparticles, which entails a clear benefit over conventional Galenic formulations, in which it is not possible to exercise control over the release of the associated molecule.

The term "biologically active molecule" relates to any substance which is used for treating, curing, preventing or diagnosing a disease or which is used for improving the physical and mental well-being of human beings and animals, as well as that compound intended for destroying, preventing the action of, counteracting or neutralizing any harmful organism, or any substance which is used as a cosmetic, as well as that compound intended for regenerating tissues or in tissue engineering. The nanoparticles object of the present invention are suitable for associating bioactive molecules regardless of the solubility characteristics thereof. The capacity for associating will depend on the corresponding molecule, but in general terms it will be high for both hydrophilic molecules and for molecules having a pronounced hydrophobic character. In a particular embodiment, the bioactive molecule is selected from peptides, proteins, lipid or lipophilic compounds, saccharide compounds, nucleic acid compounds or nucleotides such as oligonucleotides, polynucleotides or combinations of the aforementioned molecules.

In a preferred embodiment of the invention, the biologically active molecule is a peptide, protein or a bioactive molecule of cosmetic and regenerative interest, such as kinetin, or a nucleic acid derivative, such as a DNA plasmid, oligonucleotide, interfering RNA or a polynucleotide. The DNA plasmid is that which incorporates genetic material to be introduced in cells and to express proteins or that which acts as an RNA precursor.

Kinetin is a type of cytokinin, a class of plant hormones that promote cell division and differentiation.

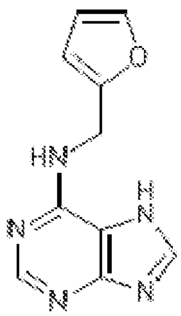

Its structure derives from an adenine with a side chain bound to the amine group in position 6 corresponding to $N^6$-furfuryladenine.

Kinetin has antioxidants and anti-aging properties and for these reasons it is used in anti-aging treatments.

The proportion of bioactive molecule associated with the nanoparticles can reach up to 95% by weight with respect to the total weight of the components of the nanoparticles. However, the suitable proportion will depend on the bioactive molecule to be incorporated, the indication for which it is used and the administration efficiency in each case. In a particular embodiment, the proportion of bioactive molecule is between 1 and 10% by weight.

In the specific case of incorporating a polynucleotide such as a DNA plasmid or a interfering RNA as the active ingredient, the proportion thereof in said system will be between 1% and 95% by weight, preferably between 1 and 30%, more preferably between 1% and 5%, even more preferably, 1%, 2.5% and 5%.

In another particular embodiment, the nanoparticle system of the present invention additionally comprises at least one compound capable of facilitating the tracking of said nanoparticles after their application into a living being. Preferably, said compound is a marker such as a membrane antigen or a staining agent such as for example fluorescein or TexasRed.

In another particular embodiment, the nanoparticle system of the invention further comprises at least one compound capable of facilitating or strengthening the effect of the biologically active molecule, such as for example an adjuvant or an immunomodulator (immunosuppresor or immunostimulator). The nanoparticle system can also be associated with a compound capable of interacting with biological components such as an antibody, an aptamer or a compound with affinity for a receptor in living beings.

In another particular embodiment, the nanoparticle system of the invention additionally comprises a stabilizing compound of lipid, fat or oily type, saccharide type, an amino acid or protein derivative, an ethylene oxide derivative or a morpholine type compound.

All the aforementioned compounds which can be associated with the nanoparticle system of the invention can be added into the solutions of the constituent polymers of the nanoparticles prior to the formation thereof or they can be added to the nanoparticles once formed.

In an additional aspect, the present invention relates to a pharmaceutical composition comprising the nanoparticle system described above.

The pharmaceutical compositions according to the invention include any liquid composition (i.e., suspension or dispersion of the nanoparticles of the invention) for application through oral route, buccal route, sublingual route, topical route, ocular route, nasal route, pulmonary route, auricular route, vaginal route, intrauterine route, rectal route, enteral route or parenteral route, or any composition in the form of a gel, ointment, cream or balm for administration through the topical route, ocular route, nasal route, vaginal route or rectal route.

In a particular embodiment, the composition is administered through oral route. In this case, the nanoparticles have the additional advantage of being stable in gastrointestinal fluids, therefore they can reach the intestinal epithelial tissue without suffering any degradation and release therein the active ingredient.

Due to their good properties for the administration on or through the skin and their lasting stability, the nanoparticle system of the invention is also suitable for cosmetic applications. Therefore, in another aspect, the invention relates to a cosmetic composition comprising the aforementioned nanoparticle system.

The cosmetic compositions according to the invention include any liquid composition (suspension or dispersion of nanoparticles) or any composition comprising the system of the invention and which is in the forma of a gel, cream, ointment or balm for administration through the topical route.

Said cosmetic composition can be applied on various surfaces of the human or animal body such as the skin, pilous and capillary system, nails, lips and external genital organs, and on the teeth or mucous membranes of the human or animal body.

In a particular embodiment of the invention, the composition comprising the system of the invention has a personal hygiene purpose, or has the purpose of perfuming, modifying the appearance of the body surface and/or correcting body odors and/or protecting or keeping it in good condition.

In a variant of the invention, the cosmetic or personal hygiene composition can also incorporate active molecules of lipophilic or hydrophilic nature which, although they do not have any therapeutic effect, have properties as a cosmetic or personal hygiene agent. Emollient agents, preservatives, fragrance substances, anti-acne agents, antifungal agents, antioxidants, deodorants, antiperspirants, anti dandruff agents, depigmenting agents, antiseborrheic agents, dyes, tanning lotions, UV light absorbers, enzymes, among others are the active molecules which can be incorporated in the nanoparticles.

In another aspect, the invention relates to a nutritional composition comprising the aforementioned nanoparticle system. Said nutritional composition can be a food, a dietary supplement or a nutritional supplement. The nutritional compositions can include milk, yoghurts, fruit and vegetable juices, desserts, infant products or dehydrated products. The nanoparticles are added to the nutritional composition by means of mixing and homogenizing according to the technical method for preparing each product. Other components such as vitamins can be additionally added to the nutritional composition. Examples of these compounds are vitamins from the group A, group B, group C, group D, group E, the folic acid group or mixtures thereof.

In another aspect, the present invention relates to a method for preparing a nanoparticle system as has been previously defined which comprises:

a) preparing an aqueous solution of at least one anionic polymer;
b) preparing a solution of a cationic cross-linking agent and, optionally adding to said solution a cationic polymer;
c) mixing the solutions obtained in a) and b) under stirring with spontaneous formation of the nanoparticles.

In a variant of the method, the cationic polymer is added to the already formed nanoparticles instead of being incorporated in the cross-linking agent solution.

The anionic polymer(s) are incorporated by means of their aqueous dissolution at a concentration between 0.1 and 6 mg/mL, more preferably between 0.1 and 5 mg/mL and yet more preferably between 0.1 and 3 mg/mL.

According to another particular embodiment, the cationic cross-linking agent is dissolved in water at a concentration between 0.0625 and 2 mg/mL, preferably between 0.25 and 2 mg/mL.

The formation of the nanoparticles object of the present invention is a consequence of a controlled ionotropic cross-linking process of the components having opposite charge. As a result of said controlled process, which is known

as ionic or ionotropic cross-linking process, homogenous, adjustable and reproducible nanoparticles having predetermined size and surface charge are obtained regardless of whether or not any bioactive molecule is associated and regardless of the electric charge which is present.

The biologically active molecule, and/or the compound capable of facilitating the tracking of the nanoparticles after their application into a living being, and/or the compound capable of facilitating or strengthening the effect of the biologically active molecule, and/or the compound capable of interacting with biological components or with affinity for a receptor in living beings, and/or the stabilizing compound, or the bioactive molecule acting as a cosmetic agent, is dissolved in one of the solutions a) or b), depending on its charge, i.e., if it has a negative charge it is dissolved in solution a) and, if in contrast, it has a positive charge, it is dissolved in solution b). In a variant of the method, said molecule is added on the nanoparticles once it is formed.

In the case of lipophilic molecules, they can first be dissolved in a small volume of an organic solvent, an oil or lipid or lipophilic compound, or a mixture of water and the aforementioned compounds, which will then be added to one of the aforementioned aqueous solutions, such that the concentration by weight of the organic solvent in the final solution is always less than 95%. In such case, the organic solvent must be extracted from the system unless it is pharmaceutically acceptable.

The method for preparing the mentioned nanoparticles can include an additional lyophilization step for the purpose of preserving them during their storage so that their initial characteristics are conserved and the volumes of product to be handled are reduced. In addition, the degree of cross-linking of the nanoparticles can be increased with this process because an approximation between the polymer chains which would facilitate increasing the degree of polymer cross-linking, as well as strengthening the effect of the cross-linking agent, can take place. For lyophilizing the nanoparticles, it may only be necessary to add small amounts of sugars such as glucose, sucrose or trehalose at a concentration ranging from 1 to 5% or other molecules acting as cryoprotectors and/or lyoprotectors. The nanoparticles of the invention have the additional advantage that the particle sizes before and after lyophilization are not significantly modified. In other words, the nanoparticles have the advantage that they can be lyophilized and resuspended without any alteration in their characteristics.

Due to the high potential of the nanoparticulate systems of the present invention in the biomedical field, said systems are suitable for treating or preventing diseases in human beings and animals for the purpose of restoring, correcting or modifying the physiological functions by exerting a pharmacological, immunological, metabolic or gene expression modifying action, or for the purpose of establishing a medical or veterinary diagnosis.

Therefore, an additional aspect of the present invention relates to the use of a nanoparticle system as previously defined in the preparation of a medicinal product.

In a particular embodiment, the systems of the invention are suitable for transferring *in vivo* or *ex vivo* a prophylactic gene for diagnosis or therapy, such as a nucleic acid fragment or an interfering RNA, into human/animal cells or primary or modified cell cultures. Therefore, the nanoparticle system of the invention is useful in the preparation of a medicinal product intended for gene therapy, gene silencing or genetic interference, or genetic vaccination.

In another particular embodiment, the nanoparticulate systems of the invention allow exploiting or altering the biological characteristics of living cells including autologous cells, allogeneic cells, xenogeneic cells or cell cultures, and subsequently using said cells or cell groups to obtain a therapeutic effect, preventive effect or for regenerative purposes, diagnosis or for modifying the production of compounds by said cells for the purpose of biotechnology production. In a particular embodiment, said manipulation includes expanding or activating cell populations *ex vivo* and adapting the cells to associate them effectively to health products used *ex vivo* or *in vivo*, such as biodegradable or non-biodegradable intrinsic microparticles or microcapsules, arrays and scaffolds.

In an additional aspect, the nanoparticle system of the invention allows modifying, correcting or introducing organoleptic properties or improving the stability in a medicinal product or in a cosmetic product.

In another additional aspect, the nanoparticulate systems of the invention allow treating, conditioning, modifying or restoring the characteristics of water, foods or nutritional supplements, as well as modifying, correcting or introducing new organoleptic properties or improving the stability thereof and facilitating or making the administration of foods or nutrients into living beings possible.

To better understand the features and advantages of the present invention, reference will be made below to a series of examples which complete the above description in an explanatory and non-limiting manner.

## Examples

As a common method for the examples described below, the nanoparticles have been characterized from the point of view of size, zeta potential (or surface charge), morphology and encapsulation efficacy.

During the explanation of some of the following examples, results obtained by means of the following techniques are referred to:

The particle size has been determined by means of the photon correlation spectroscopy technique (PCS) and

making use, to that end, of a Zeta Sizer (Zeta Sizer, Nano series, Nano-ZS, Malvern Instruments, UK) obtaining the average population size and the polydispersion index thereof. To that end, the samples were conveniently diluted in milli-Q water.

The zeta potential of the particles has been determined by means of the Laser Doppler Anemometry (LDA) technique making use, to that end, of a Zeta Sizer (Zeta Sizer, Nano series, Nano-ZS, Malvern Instruments, UK). To that end, the samples were conveniently diluted in a millimolar solution of KCl.

The association efficacy of genetic material to the nanoparticles has been determined by means of agarose gel electrophoresis technique. To that end, 1% agarose gel in TAE buffer (Tris-Acetate-EDTA, 40 mM Tris, 1% acetic acid, 1 mM EDTA) pH 8 was prepared with ethidium bromide (10 mg/mL, 5 μL) and a loading buffer and migration marker made up of glycerin (30%), bromophenol blue (0.25%) and xylene cyanol (0.25%) was used. A potential difference of 100 mV was applied for 30 minutes and a free genetic material was used as control.

The association efficacy of kinetin to the nanoparticles has been determined by means of a spectrophotometry technique. To that end, the kinetin associated with the nanoparticles of the free kinetin was separated into different formulations by means of ultrafiltration membranes (AmiconUltra 5000 MWCO, Milipore, Ireland) in a centrifuge (Beckman CR412, Beckman Coulter, US) (11,000 rcf, 30 minutes). The free kinetin was quantified at $\lambda$=265 nm against the corresponding calibration curve (y=0.0706x + 0.0012) and, for comparison, the association efficacy of the free kinetin to the nanoparticles was determined.

For performing the study of kinetin release for kinetin associated with the nanoparticles, these were incubated at 37°C in different mediums (HEPES buffer pH 7.4, acetate buffer pH 5.5, 0.01N HCl pH 2). The kinetin released at different times was determined according to the previously described methodology.

For the experiments with cell cultures, W3T3 immortalized fibroblasts (not transformed) from mice yielded by Professor Anxo Vidal from the Physiology Department of the Universidad de Santiago de Compostela (USC), were used. The fibroblasts were cultured in high glucose DMEM medium supplemented with 10% fetal bovine serum (FBS), streptomycin (0.1 mg/mL) and penicillin (100 U/mL) and 2 mM of L-glutamine (Invitrogen, SP). The cells were kept at 37°C under humidified atmosphere of 5% of $CO_2$.

The following polymers, such as those used in the following examples, were acquired from different commercial companies: Hyaluronic acid or hyaluronic (Bioibérica, Spain), colominic acid (Sigma, Spain), chondroitin sulfate (Calbiochem, USA), dextran sulfate, (Sigma, Spain), heparin (Sigma, Spain), glucomannan (Shimizu Chemical, Japan) and carrageenan (FMC Biopolymer, ME, USA), poly-L-arginine (Sigma, Spain).

The type A gelatin with a molecular weight of 238 kDa was acquired from Kerala Chemicals and Proteins (Cochim, India).

The kinetin was acquired from Sigma (Spain).

The albumin associated with some of the nanosystems as active ingredient was bovine serum albumin (BSA) acquired from Sigma (Spain).

The albumin used as a biomaterial forming the nanoparticles was human recombinant albumin yielded by Novozymes Biopharma (Nothingham, UK) and subsequently subjected to a cationization process.

The proteins (different molecular weight albumin and gelatins) cationized with spermine or ethylenediamine have been synthesized according to the method described by Seki et al. (Journal of Pharmaceutical Sciences 2006, 95 (6), 1393-1401). To that end, a 1% w/v (100 mg of protein in 10 ml of 0.1 M phosphate buffer, pH 5.3) solution of the corresponding protein was prepared, 1620 mg of spermine or 574 mg of ethylenediamine and 267.5 mg of N-(3-dimethylaminopropyl)-N'-ethyl-carbodiimide hydrochloride (EDC) were added therein. The pH (pH=4.5) was then adjusted to a value pH=5 with NaOH and the mixture was left to react for 18 hours in a thermostated water bath at 37±1°C. The mixture was subsequently dialyzed and lyophilized, thus obtaining the cationized protein with spermine or ethylenediamine which was used in the experiments that are described in the corresponding examples.

The determination of the isoionic or isoelectric point consists of measuring the concentration of hydrogen ions of a polymer solution that has been demineralized by means of contact with ion-exchange resins (Commission on Methods for Testing Photographic Gelatin. PAGI METHOD 10th Ed. 2006). The process consists of contacting a 1% solution of the cationized protein with a mixture of acid cation resin and basic anion resin at a ratio of 1:2. These exchange resins were first treated by means of washing with milliQ water at 35°C, they were then contacted with the modified protein solution under stirring at 35°C for 30 minutes. The solution was then dried and the pH was measured at 35°C. The value obtained indicates the isoionic or isoelectric point of the protein. Commercial gelatins having isoelectric points of 9 and 5 acquired from Nitta Gelatin (Ontario, Canada) and from Kerala Chemicals and Protein (Cochim, India), respectively, or human recombinant albumin have been used as control.

The DNA plasmid pEGFP was acquired from Elim Biopharmaceuticals (CA, USA).

The interfering RNA (siRNA) siGAPDH and siEGFP were acquired from Ambion (USA) and Invitrogen (USA), respectively.

The spermine and the spermidine were acquired from Sigma Aldrich (Spain).

Example 1: Preparation of nanoparticles based on dextran sulfate associating a bioactive molecule (DNA plasmid)

Nanoparticles were prepared from dextran sulfate according to the aforementioned method. A bioactive hydrophilic macromolecule was incorporated in the composition thereof, selecting for this purpose genetic material, specifically the plasmid, pEGFP. It is a negatively charged macromolecule so it was incorporated together with dextran sulfate, which also has a negative charge, to prevent the occurrence of interactions prior to the formation of the particles. The cationic spermine molecule was used as the cross-linking agent.

To that end, aqueous solutions of dextran sulfate (2 mg/mL) in milli-Q water were prepared. An aqueous solution of spermine (0.6-0.8 mg/mL) in milli-Q water was used as the cross-linking agent. The corresponding genetic material was incorporated at a proportion of 2.5% by mass. The bioactive molecule was incorporated to the solution of dextran sulfate and the resulting solution was mixed with the cross-linking solution under magnetic stirring, which was maintained for half an hour, allowing the complete evolution of the system towards a stable nanoparticulate form. The ratios between the polymer and the cross-linker are shown in Table 1. Said table also shows the average diameter and surface electric charge (zeta potential) of the systems obtained.

Table 1. Physicochemical characteristics of nanoparticles prepared from dextran sulfate (DS) associating a DNA plasmid (pEGFP).

| DS:cross-linking ratio | Cross-linker used | pDNA content (%) | Size (nm) | Polydisp. | Z Potential (mV) |
|---|---|---|---|---|---|
| 2/0.75 | Spermine | 2.5 | 171+1 | 0.11 | -13.1±0.3 |
| 2/0.6 | Spermine | 2.5 | 163±2 | 0.26 | -10.7±0.1 |

Example 2. Modulation of the surface electric charge of nanoparticles prepared from dextran sulfate associating a bioactive molecule by means of combining with another anionic polymer: Combination of dextran sulfate and chondroitin sulfate and the association of a bioactive molecule (protein)

Dextran sulfate nanoparticles were prepared according to the aforementioned method but by adding an anionic polymer excipient, chondroitin sulfate, for the purpose of modulating the characteristics of the nanoparticles, specifically the surface electric charge. A bioactive molecule was further incorporated in the composition, selecting for this purpose a protein, specifically albumin. It is a negatively charged macromolecule, so it was incorporated together with dextran sulfate, which also has a negative charge, to prevent the occurrence of interactions prior to the formation of the particles. Cationic spermidine was used as the cross-linking agent.

To that end, solutions of dextran sulfate (5 mg/mL) and chondroitin sulfate (6 mg/mL), solutions of spermidine (2 mg/mL) and albumin (5 mg/mL) in 100 mM HEPES buffer pH 7.4 were prepared. All the components with a negative charge were mixed and the bioactive molecule albumin was incorporated, giving rise to a dextran sulfate:albumin: chondroitin sulfate ratio by mass of 1:1:0.72. The resulting solution was mixed with 0.6 mL solution of spermidine under magnetic stirring, allowing the complete evolution of the system towards a stable nanoparticulate form. Thus, nanoparticles having an average particle size of 189±27 nm (polydispersion index of 0.202) and with a negative surface electric charge of -38.1±2.4 mV were prepared.

Example 3: Preparation of nanoparticles based on heparin and their association to an active ingredient.

Heparin nanoparticles were prepared according to the aforementioned method.

A bioactive hydrophilic macromolecule was incorporated in the composition thereof, selecting for this purpose genetic material, specifically the plasmid, pEGFP, or interfering RNA (siRNA), the siGAPDH. They are negatively charged macromolecules in both cases, so they were incorporated together with the heparin, which also has a negative charge, to prevent the occurrence of interactions prior to the formation of the particles. To that end, aqueous solutions of heparin (1 mg/mL) in milli-Q water were prepared. An aqueous solution of spermine (0.75 mg/mL) in milli-Q water was used as the cross-linking agent. The corresponding genetic material was incorporated at a proportion of 5% by mass. The bioactive molecule was incorporated to the solution of heparin and the resulting solution was mixed with the cross-linking solution under magnetic stirring, which was maintained for half an hour, allowing the complete evolution of the system towards a stable nanoparticulate form. Table 2 shows the average diameter and surface electric charge (zeta potential) of the systems obtained.

Table 2. Physicochemical characteristics of nanoparticles prepared from heparin (HEP) associating genetic material. (Cross-linking agent: Spermine (SPM)).

| Mass ratio (HEP:SPM) | pDNA (%) | siRNA (%) | Diameter (nm) | IPD | Zeta potential (mV) |
|---|---|---|---|---|---|
| 1:0.375 | 5 | - | 197±2 | 0.07 | -22,9±0,2 |
| 1:0.375 | - | 5 | 126±0,5 | 0.09 | -21,7±1,3 |

Example 4: Preparation of nanoparticles based on carrageenan and their association with an active ingredient.

Carrageenan nanoparticles were prepared according to the aforementioned method. A bioactive hydrophilic macromolecule was incorporated in the composition thereof, selecting for this purpose genetic material, specifically the plasmid, pEGFP. It is a negatively charged macromolecule, so it was incorporated together with carrageenan, which also has a negative charge, to prevent the occurrence of interactions prior to the formation of the particles. The cationic spermine molecule was used as the cross-linking agent. To that end, aqueous solutions of X-carrageenan (0.5 mg/mL) in milli-Q water were prepared. An aqueous solution of spermine (0.25 mg/mL) in milli-Q water was used as the cross-linking agent. The corresponding genetic material was incorporated at a proportion of 5% by mass. The bioactive molecule was incorporated to the carrageenan solution and the resulting solution was mixed with the cross-linking solution under magnetic stirring, which was maintained for half an hour, allowing the complete evolution of the system towards a stable nanoparticulate form. The average diameter of the nanoparticles obtained is 136±0.3 nm (polydispersion index of 0.23) and their surface electric charge (zeta potential) is - 28.1±1.9 mV.

Example 5: Preparation of nanoparticles based on colominic acid and their association with an active ingredient.

Colominic acid nanoparticles were prepared according to the aforementioned method. A hydrophilic macromolecule was associated with them in the composition thereof, selecting for this purpose genetic material, specifically plasmid pEGFP. It is a negatively charged bioactive macromolecule, so it was incorporated together with colominic acid to prevent the occurrence of interactions prior to the formation of the particles.

To that end, an aqueous solution of colominic acid (1 mg/mL) in milli-Q water was prepared. An aqueous solution of spermine (1.5 mg/mL) in milli-Q water was used as the cross-linking agent. The corresponding genetic material was incorporated to the solution of colominic acid at a proportion of 5% by weight with respect to the aforementioned molecules. After mixing the mentioned solutions, nanoparticles having an average particle size of 702±20 nm (polydispersion index of 0.30) and with a negative surface electric charge of -11.0±0.3 mV were obtained.

Example 6: The nanoparticles prepared from colominic acid containing an active ingredient have a regular spherical shape and a homogenous nanometric particle size: Morphological characterization of nanoparticles prepared from colominic acid associating a DNA plasmid.

Nanoparticles containing genetic material in the form of plasmid pEGFP (2.5% load) were prepared from colominic acid according to the aforementioned method. The systems were morphologically characterized by means of transmission microscopy (TEM) (CM12, Philips, Eindhoven, The Netherlands) using 1% phosphotungstic acid as the contrast agent. Figure 1 shows the corresponding images. It can be seen from said images that the nanoparticle systems have a regular spherical shape and a homogenous nanometric particle size.

Example 7. Preparation of nanoparticles based on hyaluronic acid associating a bioactive molecule (DNA plasmid).

Hyaluronic acid nanoparticles were prepared according to the aforementioned method using spermine as the ionic cross-linking agent. A hydrophilic bioactive macromolecule was associated in the composition thereof, selecting for this purpose genetic material, specifically plasmid pEGFP. It is a negatively charged macromolecule, so it was incorporated together with hyaluronic, which also has a negative charge, to prevent the occurrence of interactions prior to the formation of the particles. The cationic spermine molecule was used as the cross-linking agent. To that end, aqueous solutions in milli-Q water of hyaluronic acid (2 mg/mL) and of dissolved spermine (0.75 mg/mL) were prepared. The corresponding genetic material was incorporated at a proportion of 2.5% by weight with respect to the previous components. The bioactive molecule was incorporated to the solution of hyaluronic and the resulting solution was mixed with the spermine solution under magnetic stirring, allowing the complete evolution of the system towards a stable nanoparticulate form. Thus, nanoparticles having an average particle size of 532+21 nm (polydispersion index of 0.34) and with a negative surface electric charge of -21.1±0.1 mV were obtained.

Example 8. The nanoparticles prepared from hyaluronic acid effectively associating with bioactive molecule (siRNA) and have a regular spherical shape and a homogenous nanometric particle size

Hyaluronic acid nanoparticles associating interfering RNA, siGAPDH, were prepared according to the aforementioned method using spermine as the cross-linking agent and using the formulation conditions described in the preceding example with the exception of the genetic material content (proportion of 5% by weight with respect to the components). The association of the genetic material with the developed nanoparticles was determined by means of agarose gel electrophoresis. As seen in Figure 2-B, unlike the free siRNA control, the bands corresponding to the siRNA incorporated in the preparation of the nanoparticles do not migrate in the gel, which indicates that it is effectively associated with the nanoparticles.

In addition, the systems were morphologically characterized by means of transmission microscopy (TEM) (CM12, Philips, Eindhoven, The Netherlands) using 1% phosphotungstic acid as the contrast agent. Figure 3 shows the corresponding images. It can be seen from said images that the nanoparticle systems have a regular spherical shape and a homogenous nanometric particle size.

Example 9. Preparation of nanoparticles based on hyaluronic acid associating a bioactive molecule for cosmetic use

Hyaluronic acid nanoparticles were prepared according to the aforementioned method using spermine as the ionic cross-linking agent. A bioactive molecule was associated in the composition thereof, selecting for this purpose a cytokinin for cosmetic use, specifically $N^6$-furfuryladenine (kinetin). It is a positively charged molecule in the formation conditions of the nanoparticles, so it was incorporated together with the ionic cross-linking agent, spermine, which also has a positive charge, to prevent the occurrence of interactions prior to the formation of the particles. To that end, aqueous solutions of hyaluronic acid in acetate buffer (15 mM, pH 5.5) were prepared at a concentration of 4.5 mg/mL. Spermine dissolved in milli-Q water at a concentration of 1.125 mg/mL was used as the cross-linking agent. Kinetin was incorporated at a proportion of 5 and 10% by weight with respect to the previous components, for which purpose it was first dissolved in 0.1 N HCl and was incorporated to the cross-linking agent solution. The resulting solution was mixed with the hyaluronic acid solution under magnetic stirring, allowing the complete evolution of the system towards a stable nanoparticulate form. Table 3 shows the average diameter, surface electric charge (zeta potential) and the association efficacy of the systems obtained.

Table 3. Physicochemical characteristics of the nanoparticles prepared from hyaluronic acid (HA) using spermine (SPM) as the ionic cross-linking agent and associating a bioactive molecule for cosmetic use, kinetin (n=3).

| HA:SPM ratio | Theoretical kinetin (%) | Size (nm) | Zeta potential (mV) | Association efficacy (%) |
|---|---|---|---|---|
| 8:1 | 5 | 472±20 | -17.5±0.3 | 40±6 |
| 8:1 | 10 | 312±15 | -12.6±0.4 | 16±3 |

Example 10. The nanoparticles prepared from hyaluronic acid associating a bioactive molecule for cosmetic use have a regular spherical shape and a nanometric size

Nanoparticles associating kinetin (at a proportion of 5% by weight with respect to the previous components) were prepared from hyaluronic acid using spermine as the cross-linking agent according to the aforementioned method, The systems were morphologically characterized by means of transmission microscopy (TEM) (CM12, Philips, Eindhoven, The Netherlands) using 1% phosphotungstic acid as the contrast agent. Figure 4 shows the corresponding images. It can be seen from said images that the nanoparticle systems have a regular spherical shape and a nanometric size.

Example 11. Preparation of nanoparticles based on chondroitin sulfate associating a bioactive molecule for cosmetic use.

Chondroitin sulfate nanoparticles were prepared according to the aforementioned method using spermine as the ionic cross-linking agent. A bioactive molecule was associated in the composition thereof, selecting for this purpose a cytokinin for cosmetic use, specifically kinetin. It is a positively charged molecule in the formation conditions of the nanoparticles, so it was incorporated together with ionic cross-linking agent, spermine, which also has a positive charge, to prevent the occurrence of interactions prior to the formation of the particles.

To that end, aqueous solutions of chondroitin sulfate in acetate buffer (15 mM, pH 5.5) were prepared at a

concentration of 1.5 mg/mL. Spermine dissolved in milli-Q water at a concentration of 0.375 mg/mL was used as the cross-linking agent. Kinetin was incorporated at a proportion of 2.5 and 5% by weight with respect to the previous components, for which purpose it was first dissolved in 0.1N HCL and was incorporated to the cross-linking agent solution. The resulting solution was mixed with the chondroitin sulfate solution under magnetic stirring, allowing the complete evolution of the system towards a stable nanoparticulate form. Table 4 shows the average diameter, surface electric charge (zeta potential) and the association efficacy of the systems obtained.

Table 4. Physicochemical characteristics of the nanoparticles prepared from chondroitin sulfate (ChS) using spermine (SPM) as the ionic cross-linking agent and associating a bioactive molecule for cosmetic use, kinetin (n=3).

| ChS:SPM ratio | Theoretical Kinetin (%) | Size (nm) | Zeta potential (mV) | Association efficacy (%) |
|---|---|---|---|---|
| 8:1 | 2.5 | 209±6 | -18.2±0.1 | > 95 |
| 8:1 | 5 | 263±10 | -16.6±0.1 | > 95 |

Example 12. The nanoparticles prepared from chondroitin sulfate associating a bioactive molecule for cosmetic use have a regular spherical shape and a nanometric size

Nanoparticles associating kinetin (at a proportion of 2.5% by weight with respect to the previous components) were prepared from chondroitin sulfate according to the aforementioned method using spermine as the cross-linking agent. The systems were morphologically characterized by means of transmission microscopy (TEM) (CM12, Philips, Eindhoven, The Netherlands) using 1% phosphotungstic acid as the contrast agent. Figure 5 shows the corresponding images. It can be seen from said images that the nanoparticle systems have a regular spherical shape and a nanometric size.

Example 13. The developed nanoparticles release the associated bioactive molecule and it is possible to control said release by conveniently selecting the components of said nanoparticles.

Different nanoparticles associating kinetin (at a proportion of 5% by weight with respect to the components of the nanoparticles) were prepared according to the aforementioned method. The nanoparticle systems obtained were subjected to an *in vitro* release study in different release mediums (HEPES buffer pH 7.4, acetate buffer pH 5.5 or 0.01N HCl pH 2). Figure 6 shows the corresponding release profiles. As seen, the developed nanoparticles release the associated bioactive molecule and it is possible to control said release by conveniently selecting the components of said nanoparticles.

Example 14. Preparation of nanoparticles based on chondroitin sulfate associating a bioactive molecule for cosmetic use in cell culture medium

Chondroitin sulfate nanoparticles were prepared according to the aforementioned method using spermine as the ionic cross-linking agent. A bioactive molecule was associated in the composition thereof, selecting for this purpose a cytokinin for cosmetic use, specifically kinetin. It is a positively charged molecule in the formation conditions of the nanoparticles, so it was incorporated together with ionic cross-linking agent, spermine, which also has a positive charge, to prevent the occurrence of interactions prior to the formation of the particles.

To that end, solutions of chondroitin sulfate in 20 mM HEPES buffer pH 7.4 were prepared at a concentration of 1.0-3.0 mg/mL. Spermine dissolved in 20 mM pH 7.4 HEPES buffer at a concentration of 0.75-2.0 mg/mL was used as the cross-linking agent. Kinetin was incorporated at a proportion of 0.78-1.12% by weight with respect to the previous components, for which purpose it was first dissolved in 0.1N HCL (0.25 mg/ml) and was incorporated to the cross-linking agent solution. The resulting solution was mixed with the chondroitin sulfate solution under magnetic stirring, allowing the complete evolution of the system towards a stable nanoparticulate form.

Optionally, poly-L-arginine (PA) cationic polymers, cationized human recombinant albumin (cHSA) or different types of cationized gelatin, all of them prepared in solutions of 20 mM HEPES buffer pH 7.4 at a concentration of 0.3 mg/mL and incorporated to the cationic cross-linking agent solution, were added prior to the formation of the nanoparticles. Tables 5-10 show the average diameter of the different nanoparticle systems obtained. As seen, the incorporation of the mentioned cationic polymers allows modulating the properties of the nanoparticles. However, under the same conditions, switching one polymer for another not only significantly alters the characteristics of the nanoparticles but also causes the possibility that they are not formed or are aggregated. Therefore, it can be concluded that the use of one or another cationic polymer as the optional component in the nanoparticle systems is not common.

Table 5. Average size of the nanoparticles prepared from chondroitin sulfate (ChS), using spermine (SPM) as the ionic cross-linking agent and associating a bioactive molecule for cosmetic use, kinetin (n=3). (PDI: polydispersion index)

| ChS (mg/mL) | SPM (mg/mL) | ChS:SPM Ratio | Size (nm) | PDI |
|---|---|---|---|---|
| 3.0 | 1.5 | 7:1 | 227±5 | 0.47 |
| 2.0 | 1 .0 | 7:1 | 139±2 | 0.21 |
| 1.0 | 0.75 | 4.5:1 | 296±5 | 0.16 |
| 3.0 | 1.5 | 8:1 | 112±20 | 0.44 |
| 2.0 | 1.0 | 8:1 | 494±21 | 0.56 |
| 1.0 | 0.75 | 5.5:1 | 175±7 | 0.31 |

Table 6. Average size of the nanoparticles prepared from chondroitin sulfate (ChS) and poly-L-arginine (PA) using spermine (SPM) as the ionic cross-linking agent and associating a bioactive molecule for cosmetic use, kinetin (n=3). (PDI: polydispersion index)

| ChS (mg/mL) | SPM (mg/mL) | PA (mg/mL) | ChS:SPM Ratio | Size (nm) | PDI |
|---|---|---|---|---|---|
| 2.0 | 1.0 | 0.1 | 7:1 | 110±3 | 0.30 |
| 1.0 | 0.75 | 0.1 | 4.5:1 | 186±1 | 0.05 |
| 2.0 | 1.0 | 0.2 | 7:1 | 107±3 | 0.44 |
| 1.0 | 0.75 | 0.2 | 4.5:1 | 232±2 | 0.06 |
| 2.0 | 1.0 | 0.3 | 7:1 | 103±1 | 0.11 |
| 1.0 | 0.75 | 0.3 | 4.5:1 | 257±1 | 0.07 |

Table 7. Average size of the nanoparticles prepared from chondroitin sulfate (ChS) and human recombinant albumin cationized with spermine (cHSA-SPM) using spermine (SPM) as the ionic cross-linking agent and associating a bioactive molecule for cosmetic use, kinetin (n=3). (PDI: polydispersion index) (Concentration of cHSA-SPM is 0.3 mg/mL in all cases) (AF: Absence of formed nanoparticles) (AG: Aggregation of nanoparticles).

| ChS (mg/mL) | Concentration of SPM (mg/mL) | | | | |
|---|---|---|---|---|---|
| | **1.0** | **1.1** | **1.3** | **1.4** | **1.5** |
| **1.25** | 203±3 nm PDI 0.1 | 196±2 nm PDI 0.1 | 387±11 nm PDI 0.1 | 703±9nm PDI 0.6 | 854±160nm PDI 0.1 |
| **1.4** | AF | 180±2 nm PDI 0.1 | 260±3 nm PDI 0.1 | 420±13nm PDI 0.1 | 512±16nm PDI 0.1 |
| **1.6** | AF | 146±1 nm PDI 0.1 | 207±3 nm PDI 0.1 | 298±2 nm PDI 0.1 | 390±6nm PDI 0.1 |
| **1.8** | AF | AF | 192±3 nm PDI 0.2 | 377±4nm PDI 0.2 | 401±2 nm PDI 0.1 |

Table 8. Average size of the nanoparticles prepared from chondroitin sulfate (ChS) and gelatin cationized with ethylenediamine (GCed) using spermine (SPM) as the ionic cross-linking agent and associating a bioactive molecule for cosmetic use, kinetin (n=3). (PDI: polydispersion index) (Concentration of GCed is 0.3 mg/mL in all cases) (AF: Absence of formed nanoparticles) (AG: Aggregation of nanoparticles).

| ChS mg/mL) | Concentration of SPM (mg/mL) | | | | | |
|---|---|---|---|---|---|---|
| | 1.0 | 1.1 | 1.25 | 1.5 | 1.75 | 2.0 |
| **1.25** | 433±3 nm PDI 0.1 | 409±4 nm PDI 0.1 | 694±17 nm PDI 0.2 | AG | AG | AG |
| **1.4** | 287±3 nm PDI 0.2 | 575±5 nm PDI 0.3 | 581±30 nm PDI 0.1 | AG | AG | AG |
| **1.6** | AF | AF | AF | 316±2 nm PDI 0.4 | 706±98nm PDI 0.9 | AG |
| **1.8** | AF | AF | AF | 449±4nm PDI 0.1 | 620±8 nm PDI 0.1 | AG |

Table 9. Average size of the nanoparticles prepared from chondroitin sulfate (ChS) and gelatin cationized with spermine (GCspm) using spermine (SPM) as the ionic cross-linking agent and associating a bioactive molecule for cosmetic use, kinetin (n=3). (PDI: polydispersion index) (Concentration of GCspm is 0.3 mg/mL in all cases) (AF: Absence of formed nanoparticles) (AG: Aggregation of nanoparticles).

| ChS (mg/mL) | Concentration of SPM (mg/mL) | | | | | |
|---|---|---|---|---|---|---|
| | 1.0 | 1.1 | 1.25 | 1.5 | 1.75 | 2.0 |
| **1.25** | 224±3 nm PDI 0.1 | 260±4 nm PDI 0.1 | 520±14 nm PDI 0.2 | AG | AG | AG |
| **1.4** | 244±2 nm PDI 0.1 | 263±7 nm PDI 0.2 | 420±13 nm PDI 0.2 | AG | AG | AG |
| **1.6** | AF | AF | 350±9 nm PDI 0.2 | AG | AG | AG |
| **1.8** | AF | AF | 321±7 nm PDI 0.2 | 338±4nm PDI 0.2 | AG | AG |

Table 10. Physicochemical properties of the nanoparticles prepared from chondroitin sulfate (ChS) and gelatin cationized with spermine (GCspm) using spermine (SPM) as the ionic cross-linking agent and associating a bioactive molecule for cosmetic use, kinetin (n=3). (PDI: polydispersion index) (Concentration of GCspm is 0.3 mg/mL in all cases) (E.E.: Association efficacy of kinetin).

| Components ChS:SPM:GC | Size (nm) | PDI | Potential □ (mV) | pH | E.E. of kinetin (%) |
|---|---|---|---|---|---|
| 4.3:1:0.13 | 260±4 | 0.12 | -26±1 | 7.0 | 20 |
| 4.7:1:1.4 | 224±3 | 0.08 | -24±2 | 7.0 | 20 |
| 5:1:1.3 | 263±7 | 0.15 | -28±1 | 7.0 | 25 |

Example 15. I t is possible to effectively isolate the nanoparticles based on chondroitin sulfate by associating a bioactive molecule for cosmetic use and resuspending them without altering them

Nanoparticles from were prepared chondroitin sulfate and gelatin cationized with spermine (GCspm) in culture

medium according to the aforementioned method using spermine as the ionic cross-linking agent and associating kinetin. Nanoparticles having an average particle size of 263±7 nm (polydispersion index of 0.15) and with a negative surface electric charge of - 28±1 mV were thus obtained. For the purpose of isolating the nanoparticles from the formation medium, it was centrifuged at 5,000 rcf for 40 minutes at 4°C (Beckman CR412, Beckman Coulter, US). After the isolation thereof, the nanoparticles were resuspended in 20 mM pH 7.4 HEPES culture medium and physicochemically characterized. The results obtained were an average particle size of 279±4 nm (polydispersion index of 0.19) and a surface negative electric charge of -25±2 mV. As seen, the isolation process and the subsequent reconstruction process of the nanoparticles do not lead to alterations in their physicochemical characteristics.

Example 16. It is possible to lyophilize the nanoparticles based on chondroitin sulfate associating a bioactive molecule for cosmetic use and reconstituting them without altering them

The nanoparticles were lyophilized for the purpose of developing a more stable dosage form. To that end, the nanoparticles were prepared from chondroitin sulfate and gelatin cationized with spermine (GCspm) in culture medium according to the aforementioned method using spermine as the ionic cross-linking agent and associating kinetin. Suspensions of the nanoparticles at different concentrations (0.125-0.5 mg/ml) were lyophilized in the presence of glucose or trehalose at a final concentration of 5% (w/v). To that end, the suspensions (3 ml) were subjected to a freezing process at -35°C and subsequent lyophilization (Virtis Genesis freeze dryer, 25ES, Virtis, NY, USA). After lyophilization, the nanoparticle systems were resuspended without difficulty by means adding 3 mL of mQ water, giving rise to a suspension of nanoparticles and the average size of the nanoparticles was then determined. Figure 7 shows the particle size variation index (Df/Di = Ratio between the average particle size of the formulation before lyophilization and the average size after lyophilization and subsequent resuspension of the formulation in milliQ water). As seen, when the nanoparticles are lyophilized at a concentration of 0.5 mg/ml in the presence of 5% glucose, their particle size is not modified as they are subjected to a lyophilization process.

Example 17. The nanoparticulate systems based on chondroitin sulfate associating a bioactive molecule for cosmetic use do not present cytotoxicity in fibroblasts.

Nanoparticles were prepared from chondroitin sulfate and gelatin cationized with spermine (GCspm) in culture medium according to the aforementioned method using spermine as the ionic cross-linking agent and associating kinetin. The evaluation of the viability of the cells in contact with nanoparticles prepared from chondroitin sulfate combined with cationized gelatin was carried out in fibroblasts. To that end, the cells were seeded in Costar ® (Corning, US) 96-well plates at a confluence of 10,000 cells per well and were left to grow for 24h before the assay. The cells were incubated for 3 hours with different concentrations of nanoparticles in DMEM/F-12 medium (final volume in each well is 200 μL). After that time, the cells were washed and 200 μL of the complete culture medium were added. The cells were then incubated with 200 μL of RPMI medium without phenol red with XTT (0.2 mg/mL) for 15 hours. The results were expressed as a percentage of cell viability in relation to untreated control (negative control). As seen in Figure 8, the systems do not present significant toxicity in fibroblasts.

Example 18. The nanoparticle systems based on chondroitin sulfate associating a bioactive molecule for cosmetic use are effectively internalized in fibroblasts.

Nanoparticles were prepared from chondroitin sulfate and gelatin cationized with spermine (GCspm) in culture medium according to the aforementioned method using spermine as the ionic cross-linking agent and associating kinetin. The evaluation of the cellular internalization capacity of the nanoparticles was carried out in fibroblasts. To that end, the chondroitin sulfate was first labeled with fluoresceinamine (ChS-fl) according to the method described by De la Fuente et al. (Gene therapy, 15, 9, 2008, 668-76). Specifically, 20 mL of DMSO were added to 40 mL of an aqueous solution of CS (1.25 mg/mL) and 0.5 mL of fluoresceinamine (50 mg/mL in DMSO), 25 μL of cyclohexylisocyanide and 25 μL of acetaldehyde were then added to the previous solution. The mixture was maintained under magnetic stirring for 5h in the dark. The chondroitin sulfate thus labeled with fluoresceinamine (ChS-fl) was purified by means of salting-out, adding to that end a saturated solution of NaCl and cold ethanol. The CS-fl precipitate was resuspended in milli-Q water and subsequently lyophilized. This polymer together with gelatin cationized with spermine were used for the preparation of the nanoparticles associating kinetin according to the previously described method.

The fibroblasts were seeded at a cell density of 50,000 cells per chamber in multi-chamber slides (Nunc, Denmark). After 24 hours, 50 microliters of suspension of nanoparticles were incubated for 1 hour with the cells. Subsequently, the cells were fixed with paraformaldehyde (3.5% in PBS pH 7.4) and the cytoskeleton was stained with red bodipy 650/665 phalloidin (Molecular probes, US). The internalization was observed in a Leica TCS SP2 fluorescence confocal microscope (Leica Microsystems, Germany), in which the nanoparticles labeled with fluoresceinamine (green

glow) and the fibroblasts (red glow) as shown in Figure 9 could be seen.

Example 19: Preparation of nanoparticles prepared from chondroitin sulfate associating bioactive molecules (genetic material)

Chondroitin sulfate nanoparticles were prepared according to the aforementioned method using spermine as the ionic cross-linking agent. A hydrophilic bioactive macromolecule was associated in the composition thereof, selecting for this purpose genetic material, specifically the plasmid, pEGFP, or the interfering RNA (siRNA), siGAPDH. They are negatively charged macromolecules in both cases, so they were therefore together with chondroitin sulfate, which also has a negative charge, to prevent the occurrence of interactions prior to the formation of the particles. Cationic spermine molecule was used as the ionic cross-linking agent. To that end, aqueous solutions of chondroitin sulfate (1 mg/mL) and spermine (0.5 mg/mL) in milli-Q water were prepared. The corresponding genetic material was incorporated at a proportion of 5% by weight with respect to the previous components. The bioactive molecule was incorporated to the solution of chondroitin sulfate and the resulting solution was mixed with the solution of the spermine cross-linking agent under magnetic stirring, allowing the complete evolution of the system towards a stable nanoparticulate form. Table 11 shows the average diameter and surface electric charge (zeta potential) of the systems obtained.

Table 11. Physicochemical characteristics of the nanoparticles prepared from chondroitin sulfate (ChS) using spermine (SPM) as the ionic cross-linking agent and associating genetic material (DNA plasmid or siRNA) as the bioactive molecules.

| Ratio by weight (ChS:SPM) | pEGFP (%) | siGAPDH (%) | Diameter (nm) | IPD | Zeta potential (mV) |
|---|---|---|---|---|---|
| 1:0.25 | 5 | - | 192±1 | 0.06 | -21.9±0.3 |
| 1:0.25 | - | 5 | 136±1 | 0.05 | -15.4±1.4 |

Example 20. Modulation of the surface electric charge of nanoparticles prepared from chondroitin sulfate associating a bioactive molecule (interfering RNA) by means of adding a cationic polymer.

Nanoparticles were prepared from chondroitin sulfate according to the aforementioned method using spermine as the ionic cross-linking agent, adding a polymer excipient, the gelatin previously cationized with ethylenediamine, having a charge opposite that of chondroitin sulfate for the purpose of modulating the characteristics of the nanoparticles, specifically the surface electric charge. A bioactive hydrophilic macromolecule was further incorporated in the composition thereof, selecting for this purpose genetic material, specifically the interfering RNA (siRNA), siGAPDH. It is a negatively charged macromolecule, so it was incorporated together with chondroitin sulfate to prevent the occurrence of interactions prior to the formation of the particles. Cationic spermine molecule was used as the cross-linking agent. To that end, solutions of chondroitin sulfate (0.5 mg/mL) and of the polymer, gelatin previously cationized with ethylenediamine (2 mg/mL) intended for modulating the surface electric charge, and of spermine (0.6 mg/mL), were prepared in 100 mM pH 7.4 HEPES buffer. The corresponding genetic material was incorporated at a proportion of 5% by weight with respect to the previous components. The bioactive molecule was incorporated to the chondroitin sulfate solution and the resulting solution was mixed with the solutions of cationized gelatin and the cross-linking agent spermine under magnetic stirring, allowing the complete evolution of the system towards a stable nanoparticulate form. The average diameter of the nanoparticles obtained is 268+/-14 nm (polydispersion index of 0.18) and their surface electric charge (zeta potential) is + 34 +/- 1 mV. The association of the genetic material with the developed nanoparticles was determined by means of agarose gel electrophoresis. As seen in Figure 2-A, unlike the free siRNA control, the bands corresponding to the siRNA incorporated in the preparation of the nanoparticles do not migrate in the gel, which indicates that it is effectively associated with the nanoparticles.

Example 21. It is possible to obtain an effective biological response in human cells using nanoparticles prepared from chondroitin sulfate associating interfering RNA and with surface electric charge modulated by means of adding a cationic polymer

The nanoparticles prepared from chondroitin sulfate associating interfering RNA and with a surface electric charge modulated by means of adding a cationic polymer described in the previous example were subjected to biological evaluation. To that end, human cornea HCE (Human Corneal Epithelial) cells were used, in which the silencing capacity corresponding to glyceraldehyde-3-phosphate dehydrogenase (GAPDH) by the nanoparticles associating specific siRNA against the expression of this protein (siGAPDH) was determined using the nanoparticles associating a non-specific

siRNA against the expression of GAPGH (siEGFP) as negative control. The cells were seeded 24 hours before the experiment in Costar ® 96-well plates (Corning, USA) at a cell density of 7000 cells per well in 200 microliters of DMEM/F12 + glutamax culture medium supplemented with 15% of fetal bovine serum (FBS), streptomycin (0.1 mg/mL), penicillin (100 U/mL), Epithelial Growth Factor (EGF) (10 ng/ml), human insulin (5 micrograms/ml) (Invitrogen, SP), 0.5% DMSO (Sigma, Spain), and cholera toxin of *Vibrio Cholerae* (0.1 micrograms/ml) (Gentaurus, USA). The cells were kept this way at 37°C under humidified atmosphere of 5% $CO_2$. The cells were then incubated for 3 hours together with a suspension of the nanoparticles associating siRNA in 100 microliters of 1x HBSS, reaching siRNA concentrations of 75 nM and 150 nM (corresponding to the doses of 104 and 140 ng). The amount of expressed GAPDH was quantified after 48 hours by means of the kinetic fluorescence technique in a fluorimeter, Perkin Elmer Luminescence Spectrometer LS50B (Perkin Elmer, USA) and by making use of a commercial kit specifically made for this purpose (KDalert™ GAPDH Assay Kit, Ambion, USA). The silencing values provided by the nanoparticles associating siRNA were determined from said quantification, relating to that end the amount of protein expressed by the cells that are subjected to treatment with specific siRNA (siGAPDH) and the cells treated with non-specific siRNA (siEGFP) as the negative control. The mathematical expression used is the following:

% of silencing of GAPDH expression = [100 - (δ Fluorescence with nanoparticles associating siGAPDH/ δ Fluorescence with nanoparticles associating siEGFP)] * 100

The silencing values that were obtained are shown in Figure 10 in which an average silencing of the protein expression of 55% can be observed. The differences found compared to the values of the negative controls (cells treated with non-specific siRNA (siEGFP)) allow concluding that it is possible to obtain an effective biological response in human cells using nanoparticles prepared from chondroitin sulfate associating interfering RNA and with surface electric charge modulated by means of adding a cationic polymer. The efficacy of the systems developed to associate siRNA, to protect it against possible degradation processes, to transport the genetic material into the cell, and to release it in its site of action maintaining biological activity is deduced therefrom.

## Claims

**1.** A system for administering biologically active molecules comprising nanoparticles having an average size of less than 1 micrometer, comprising:

(a) at least one anionic polymer;
(b) a cationic cross-linking agent; and optionally
(c) a cationic polymer;

**characterized in that** the nanoparticles are cross-linked by means of electrostatic type interactions.

**2.** The system according to claim 1, wherein the anionic polymer is selected from hyaluronic acid or the salts thereof, colominic acid or derivatives, chondroitin sulfate, keratan sulfate, dextran sulfate, heparin, carrageenan and glucomannan or derivatives thereof.

**3.** The system according to any of the preceding claims, wherein the cationic cross-linking agent is an amine selected from spermine and spermidine, or the salts thereof.

**4.** The system according to any of the preceding claims, wherein the cationic polymer is selected from cationized dextrans, polyamino acids and modified proteins.

**5.** The system according to any of the preceding claims, wherein the average particle size is comprised between 1 and 999 nm, preferably between 50 and 600 nm, more preferably between 100 and 400 nm.

**6.** The system according to the preceding claims, additionally comprising at least one biologically active molecule.

**7.** The system according to claim 6, wherein the biologically active molecule is at a proportion of up to 95% by weight with respect to the total weight of the components of the nanoparticles.

**8.** The system according to claims 6 or 7, wherein the biologically active molecule is selected from peptides, proteins, lipid or lipophilic compounds, saccharide compounds, nucleic acid or nucleotide compounds and mixtures thereof.

**9.** The system according to claim 8, wherein the biologically active molecule is selected from DNA plasmid, an oligonucleotide, interference RNA and a polynucleotide.

**10.** The system according to any of the preceding claims, additionally comprising at least one compound capable of facilitating the tracking of the nanoparticles after their application into a living being.

**11.** The system according to claim 10, wherein the compound is a marker, a tracking agent or a staining agent.

**12.** The system according to claims 6 to 11, additionally comprising a compound capable of facilitating or strengthening the effect of the biologically active molecule.

**13.** The system according to claim 12, wherein the compound is an adjuvant or an immunomodulator.

**14.** The system according to any of the preceding claims, additionally comprising a compound capable of interacting with biological components or components with affinity for a receptor in living beings.

**15.** The system according to claim 14, wherein the compound is an antibody or an aptamer.

**16.** The system according to any of the preceding claims, additionally comprising a stabilizing compound of lipid, fat or oily type, saccharide type, an amino acid or protein derivative, an ethylene oxide derivative or a morpholine type compound.

**17.** The system according to any of the preceding claims, wherein the nanoparticles are in lyophilized form.

**18.** A pharmaceutical composition comprising a system as has been defined in any of claims 1 to 17.

**19.** The composition according to claim 18 for the administration through oral route, buccal route, sublingual route, topical route, ocular route, nasal route, pulmonary route, auricular route, vaginal route, intrauterine route, rectal route, enteral route or parenteral route.

**20.** A cosmetic or personal hygiene composition comprising a system as has been defined in any of claims 1 to 17.

**21.** The composition according to claim 20 for the administration on the skin, pilous and capillary system, nails, lips, external genital organs, teeth or mucous membranes.

**22.** The composition according to any of claims 20 to 21, further comprising emollient agents, preservatives, fragrance substances, anti-acne agents, antifungal agents, antioxidants, deodorants, antiperspirants, antidandruff agents, depigmenting agents, whitening agents, antiseborrheic agents, dyes, tanning lotions, UV light absorbers, or enzymes.

**23.** A nutritional composition comprising a system as has been defined in any of claims 1 to 17.

**24.** A method for preparing a system as defined in any of claims 1 to 17, which comprises:

a) preparing an aqueous solution of at least one anionic polymer;
b) preparing an aqueous solution of a cationic cross-linking agent and optionally adding therein a cationic polymer;
c) mixing the solutions obtained in a) and b) under stirring with spontaneous formation of the nanoparticles.

**25.** The method according to claim 24, wherein the cationic polymer is added on the already formed nanoparticles

**26.** The method according to claims 24 to 25, which further comprises adding a biologically active molecule, and/or a compound capable of facilitating the tracking of the nanoparticles after their application into a living being, and/or a compound capable of facilitating or strengthening the effect of the biologically active molecule, and/or a compound capable of interacting with biological components or components with affinity for a receptor in living beings, and/or a stabilizing compound, in solution a) if it is anionic or in solution b) if it is cationic.

**27.** The method according to any of claims 24 to 26, comprising an additional step after step c) in which the nanoparticles are subjected to a lyophilization process.

**28.** The method according to claim 27, comprising an additional step in which the lyophilized nanoparticles are regenerated.

**29.** Use of a system as defined in any of claims 1 to 17 in the preparation of a medicinal product.

**30.** Use according to claim 29, wherein the medicinal product is for gene therapy, gene silencing or genetic interference, or genetic vaccination.

**31.** Use of a system as defined in any of claims 1 to 17 for manipulating or altering the biological characteristics of living cells including autologous cells, allogeneic cells, xenogeneic cells or cell cultures and for subsequently using said cells or cell groups to obtain a therapeutic effect, preventive effect, diagnosis or for regenerative purposes, or for modifying the production of compounds by said cells, or for producing the expansion or activation of cell populations or for effectively adapting and associating them to microparticles or microcapsules, matrices and scaffolds.

**32.** Use of a system as defined in any of claims 1 to 17 for modifying, correcting or introducing organoleptic properties or for improving the stability in a medicinal product or in a cosmetic or personal hygiene product.

**33.** Use of a system as defined in any of claims 1 to 17 for conditioning, modifying or restoring the characteristics of water, foods or nutritional supplements, as well as for modifying, correcting or introducing new organoleptic properties or for improving the stability thereof and for facilitating or making the administration of foods or nutrients to living beings possible.

**34.** Use of a cosmetic or personal hygiene composition as defined in claims 20 to 22 for hygiene or aesthetic purposes, for neutralizing or removing ectoparasites, for perfuming, for modifying the appearance of the body surface and/or for correcting body odors and/or for protecting or keeping it in good condition.

**35.** Use of a system as defined in any of claims 1 to 17 for diagnostic purposes.

FIGURE 1

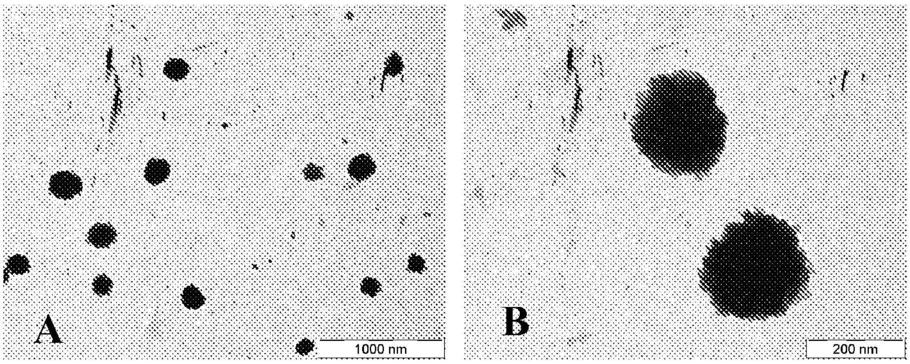


FIGURE 2

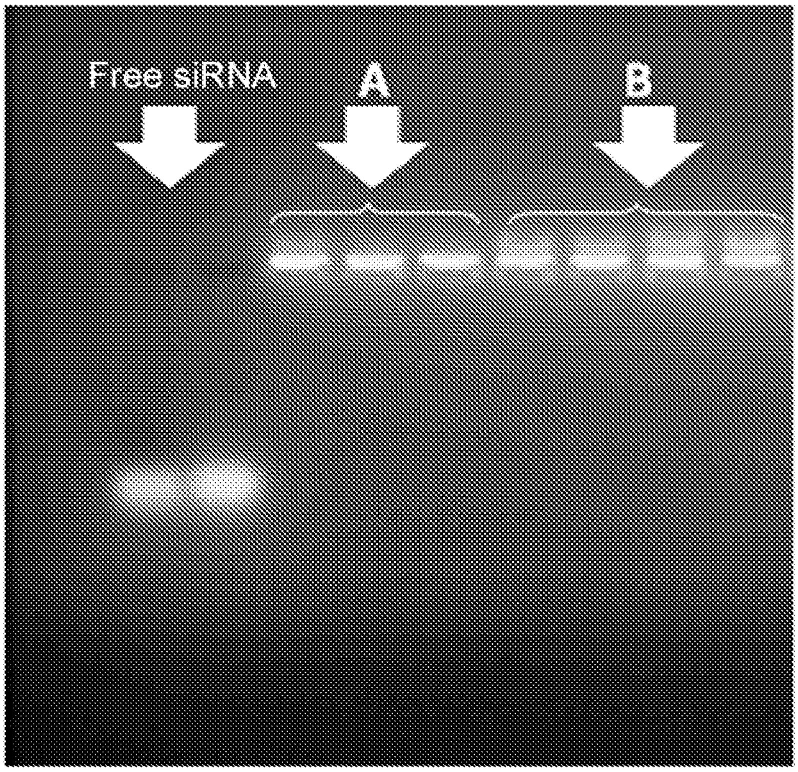

FIGURE 3

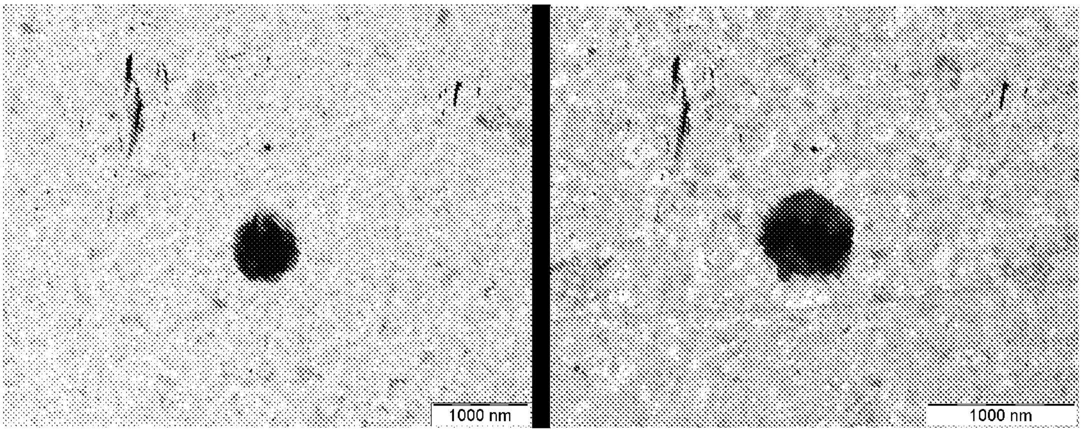


FIGURE 4

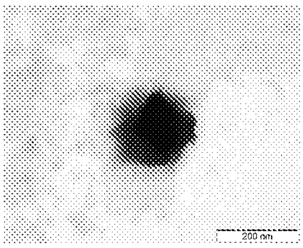


FIGURE 5

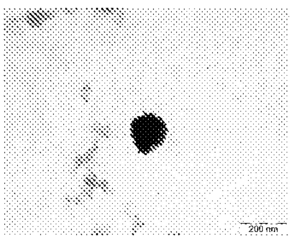

FIGURE 6 A and B

FIGURE A

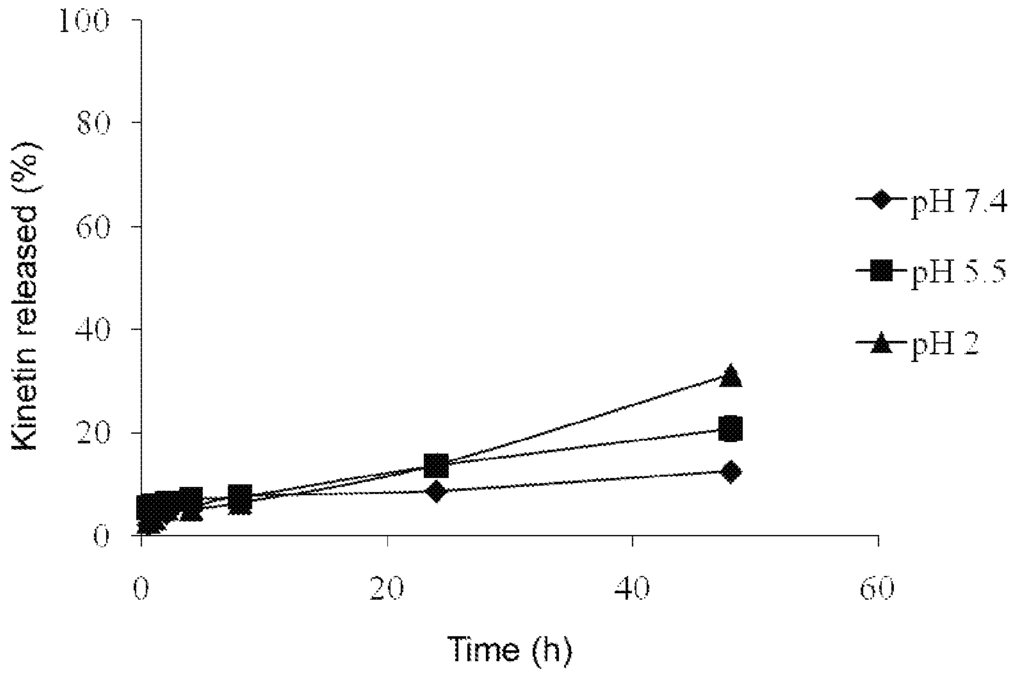


FIGURE B

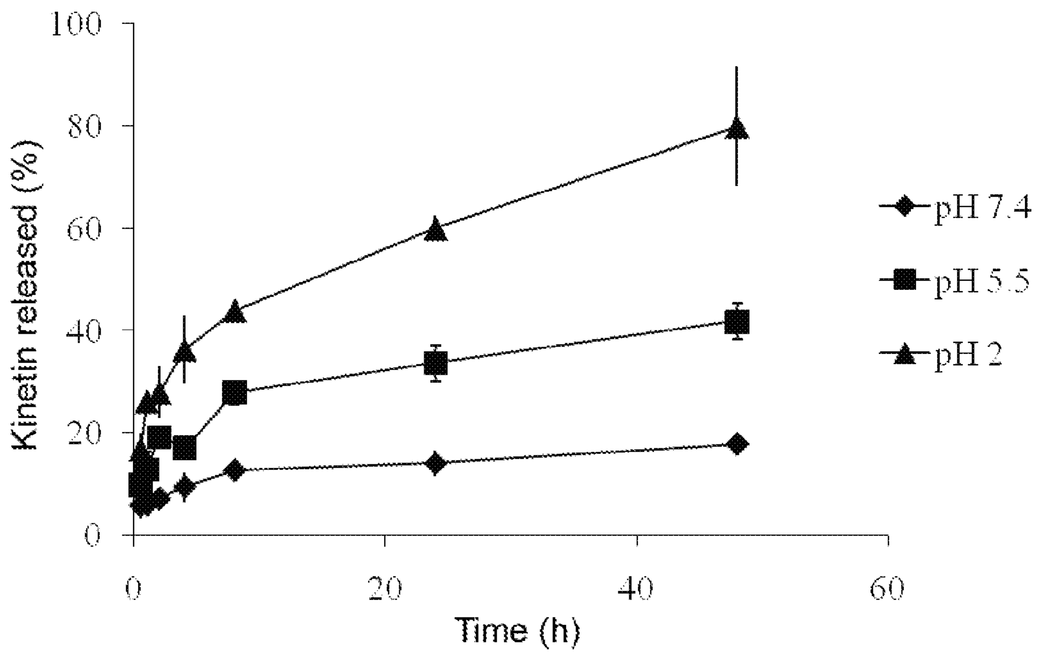

FIGURE 7

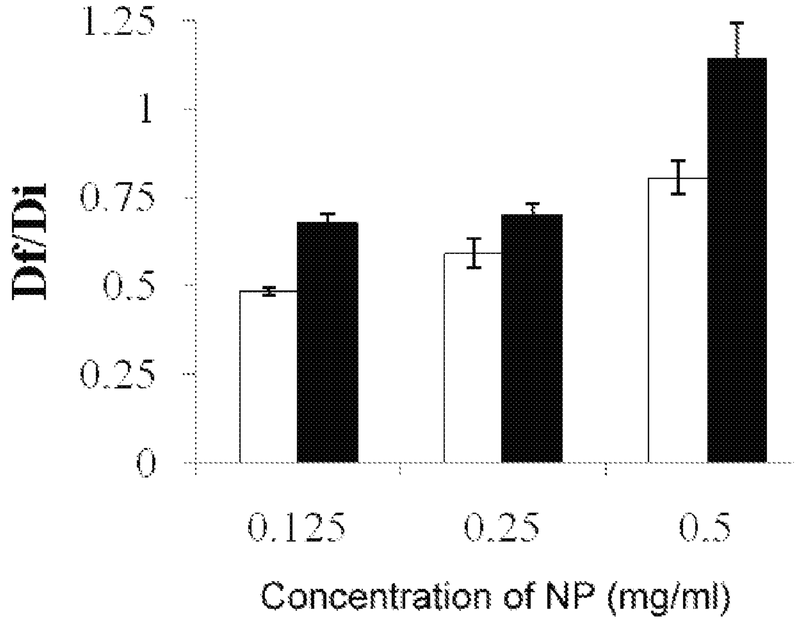


FIGURE 8.

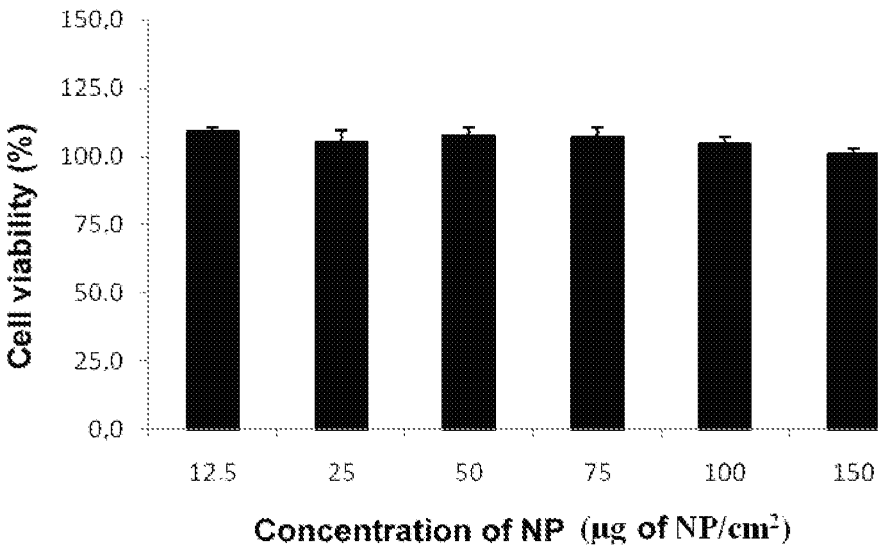

FIGURE 9

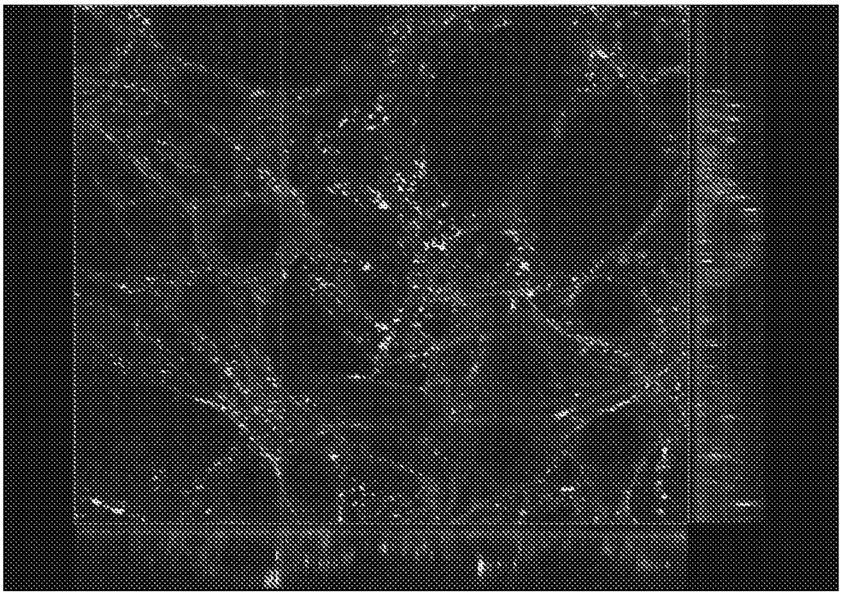

FIGURE 10

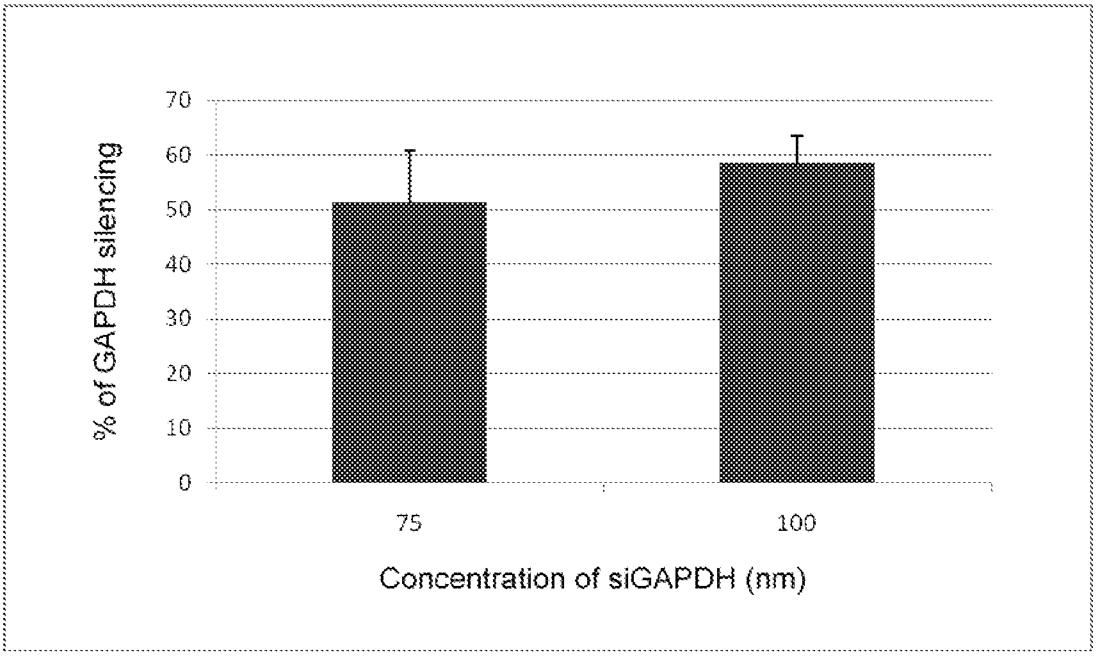

# INTERNATIONAL SEARCH REPORT

| International application No. |
|---|
| PCT/ ES 2009/070461 |

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

INVENES, EPODOC, CAS, REGISTRY, WPI, EMBASE, MEDLINE, BIOSIS, NPL

C. DOCUMENTS CONSIDERED TO BE RELEVANT

| Category* | Citation of document, with indication, where appropriate, of the relevant passages | Relevant to claim No. |
|---|---|---|
| X | US 2005008572 A1 (PROKOP et al.) 13.01.2005; paragraphs [0019]-[0044], [0063]-[0083]; example 8. | 1-19, 24-31, 35 |
| Y | | 20-23, 32-34 |
| X | WO 9918934 A1 (VANDERBILT UNIVERSITY) 22.04.1999; page 4, line 21 - page 6, line 27; examples 2-6; tab the I. | 1-19, 24-31, 35 |
| Y | | 20-23, 32-34 |
| X | US 6383478 B1 (PROKOP et al.) 07.05.2002; column 4, line 35 - column 7, line 16; example 4. | 1-19, 24-31, 35 |
| Y | | 20-23, 32-34 |

☒ Further documents are listed in the continuation of Box C. ☒ See patent family annex.

* Special categories of cited documents:

"A" document defining the general state of the art which is not considered to be of particular relevance.

"E" earlier document but published on or after the international filing date

"L" document which may throw doubts on priority claim(s) or which is cited to establish the publication date of another citation or other special reason (as specified)

"O" document referring to an oral disclosure use, exhibition, or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other documents , such combination being obvious to a person skilled in the art

"&" document member of the same patent family

| Date of the actual completion of the international search | Date of mailing of the international search report |
|---|---|
| 03 March 2010 (03.03.2010) | **(05/03/2010)** |
| Name and mailing address of the ISA/<br>O.E.P.M.<br>Paseo de la Castellana, 75 28071 Madrid, España.<br>Facsimile No. 34 91 3495304 | Authorized officer<br>N. Vera Gutiérrez<br>Telephone No. +34 91 349 55 44 |

Form PCT/ISA/210 (second sheet) (July 2009)

## INTERNATIONAL SEARCH REPORT

| International application No. |
|---|
| PCT/ES 2009/070461 |

C (continuation). DOCUMENTS CONSIDERED TO BE RELEVANT

| Category* | Citation of documents, with indication, where appropriate, of the relevant passages | Relevant to claim No. |
|---|---|---|
| X<br><br><br>Y | US 2008254078 A1 (KAUPER et al.) 16.10.2008; paragraphs [0019]-[0032], [0041]-[0045], [0053]-[0058]; examples 1-3. | 1-35<br><br><br>20-23, 32-34 |
| X<br><br><br><br><br>Y | TIYABOONCHAI, WARE et al.; Formulation and characterization of amphotericin B-chitosan-dextran sulfate nanoparticles; International Journal of Pharmaceutics 329 (2007) pages 142-149; ISSN 0378-5173. | 1-19, 24-31, 35<br><br><br><br>20-23, 32-34 |

Form PCT/ISA/210 (continuation of second sheet) (July 2009)

INTERNATIONAL SEARCH REPORT

International application No.
PCT/ES 2009/070461

**Box No. II** Observations where certain claims were found unsearchable (Continuation of item 2 of first sheet)

This international search report has not been established in respect of certain claims under Article 17(2)(a) for the following reasons:

1. [x] Claims Nos.: 31, 34, 35
because they relate to subject matter not required to be searched by this Authority, namely:
Claims 31, 34 and 35 relate to a method for treatment of the human or animal body by therapy and to a diagnostic method. The search was carried out based on the possible effects of the composition

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III** Observations where unity of invention is lacking (Continuation of item 3 of first sheet)

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

Remark on Protest

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## INTERNATIONAL SEARCH REPORT

Information on patent family members

International application No.

PCT/ ES 2009/070461

| Patent document cited in the search report | Publication date | Patent family member(s) | Publication date |
|---|---|---|---|
| US 2005008572 A | 13.01.2005 | WO 2004096998 A<br>US 2005266090 A | 11.11.2004<br>01.12.2005 |
| WO 9918934 A | 22.04.1999 | AU 9799198 A<br>EP 1021168 A<br>US 2003170313 A<br>US 6726934 B<br>US 2004136961 A | 03.05.1999<br>26.07.2000<br>11.09.2003<br>27.04.2004<br>15.07.2004 |
| US 6383478 B | 07.05.2002 | WO 0064954 A<br>AU 4476600 A | 02.11.2000<br>10.11.2000 |
| US 2008254078 A | 16.10.2008 | WO 2007031812 A<br>EP 1968613 A | 22.03.2007<br>17.09.2008 |

Form PCT/ISA/210 (patent family annex) (July 2009)

**INTERNATIONAL SEARCH REPORT**

International application No.
PCT/ ES 2009/070461

CLASSIFICATION OF SUBJECT MATTER

***A61K 9/51*** (2006.01)
***A61K 47/36*** (2006.01)
***A61K 47/18*** (2006.01)

Form PCT/ISA/210 (extra sheeet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005021044 A
- US 20077155658 A
- US 6565873 B
- US 7053034 B
- US 20050266090 A1
- US 20050008572 A1
- WO 20019620698 A1
- US 6383478 B1
- US 7045356 B
- US 6916490 B
- US 6919091 B
- US 7098032 B
- US 6475995 B
- US 7344887 B
- WO 2008033253 A


### Non-patent literature cited in the description


- The ethics and politics of nanotechnology. Division of Ethics of Science and Technology, 2006
- U.S. Food and Drug Administration. Nanotechnology, A Report of the U.S. Food and Drug Administration Nanotechnology. July 2007
- WHO, Initiative for Vaccine Research of the Department of Immunization. Vaccines and Biologicals. April 2006
- **M. Friede ; M.T. Waterdo.** *Advanced Drug Delivery Reviews,* 2005, vol. 57, 325-31
- **T.G. Park ; J.H. Jeong ; S.W. Kim.** *Advanced Drug Delivery Reviews,* 2006, vol. 58, 467-486
- **A. M. Dyer ; M. Hinchcliffe ; P. Watts ; J. Castile ; Jabbal-Gill ; R. Nankervis ; A. Smith ; L. Illum.** *Pharm. Res.,* 2002, vol. 19, 998-1008
- **B. Loretz ; A. Bernkop-Schnürch.** *Nanotoxicology,* 2007, vol. 1, 139-148
- Nanotechnology, A Report of the U.S. Food and Drug Administration Nanotechnology Task Force. U.S. Food and Drug Administration, July 2007
- **Calvo P ; Vila-Jato JL ; Alonso MJ.** *Int J Pharm.,* 1997, vol. 153, 41-50
- **Moreau et al.** *Journal of Drug Targeting,* 2002, vol. 10, 161-173
- FDA Consumer magazine. U.S. Food and Drug Administration, November 2005
- **Kainthan et al.** *Biomaterials,* 2006, vol. 27, 5377-5390
- **N. Blow.** *Nature,* 2007, vol. 450, 1117-1120
- **J.K. Vasir ; V. Labhasetwar.** *Expert Opinion on Drug Delivery,* 2006, vol. 3, 325-344
- **Q. Gana ; T. Wang ; C. Cochrane ; P. McCarron.** *Colloids and Surfaces B: Biointerfaces,* 2005, vol. 44, 65-73
- **Gregoriadis G et al.** *Cell. Mol. Life Sci.,* 2000, vol. 57, 1964-1969
- **Eisenberg et al.** *Nature Cell Biology,* 04 October 2009
- **Belting M. et al.** *Biochem J,* 1999, vol. 338, 317-323
- **Seki et al.** *Journal of Pharmaceutical Sciences,* 2006, vol. 95 (6), 1393-1401
- Commission on Methods for Testing Photographic Gelatin. PAGI METHOD. 2006
- **De la Fuente et al.** *Gene therapy,* 2008, vol. 15 (9), 668-76